# FASCICULE DE BREVET EUROPEEN

(11) **EP 1 370 230 B1**
(45) Date de publication et mention de la délivrance du brevet: **23.08.2006**
(21) Numéro de dépôt: 01271202.2
(22) Date de dépôt: 20.11.2001
(51) Int. Cl.: A61Q 17/04, A61K 8/37, A61K 8/35, A61K 8/49, A61K 8/40

(54) **COMPOSITIONS COSMETIQUES ANTISOLAIRES A BASE D'UN MELANGE SYNERGIQUE DE FILTRES ET UTILISATIONS**
KOSMETISCHE SONNENSCHUTZZUSAMMENSETZUNGEN AUF BASIS EINES SYNERGISTISCHEN FILTERGEMISCHES UND ANWENDUNGEN
COSMETIC SUNSCREEN COMPOSITIONS BASED ON A SYNERGIC MIXTURE OF FILTERS AND USES

(30) Priorité: 18.12.2000 FR 0016519
(43) Date de publication de la demande: 17.12.2003
(73) Titulaire: L'ORÉAL, 75008 Paris (FR)
(72) Inventeur: CANDAU, Didier, F-91570 Bièvres (FR)
(74) Mandataire: Miszputen, Laurent
(86) Numéro de dépôt international: PCT/FR2001/003634
(87) Numéro de publication internationale: WO 2002/049594

(56) Documents cités:
- EP-A- 0 711 778
- EP-A- 0 967 200
- EP-A- 1 008 586
- EP-A- 1 133 981
- WO-A-94/06404
- DE-A- 19 746 654
- DE-A- 19 755 649
- FR-A- 2 783 711
- FR-A- 2 795 638
- NN-A-

## Description

L'invention concerne de nouvelles compositions cosmétiques ou dermatologiques à usage topique, en particulier pour la photoprotection de la peau et/ou des cheveux, caractérisées par le fait qu'elles comprennent, dans un support cosmétiquement acceptable, au moins :
(i) au moins un dérivé silicié à fonction benzotriazole à titre de premier filtre ;
(ii) au moins un dérivé de dibenzoylméthane à titre de deuxième filtre ;
(iii) au moins un composé 4,4-diarylbutadiène à titre de troisième filtre.

L'invention concerne également leurs applications à la protection de la peau et des cheveux contre les effets du rayonnement ultraviolet.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber selectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV.

On connaît dans l'état de la technique des filtres UV cosmétiques des dérivés siliconés à fonction benzotriazole lipophiles présentant de bonnes propriétés filtrantes aussi bien dans le domaine des radiations UV-A que dans le domaine des radiations UV-B. Ils sont décrits dans les demandes EP-A-0392883 ; EP-A-0660701 ; EP-A-0708108 ; EP-A-0711778 ; EP-A-711779.

Dans les demandes EP-A-0742003 et EP-A-0860165, on a déjà proposé d'associer à ces filtres silicones à fonction benzotriazole des filtres hydrosolubles à fonction suifonique particuliers à savoir l'acide benzène 1,4-di(3-méthylidène-10-camphosulfonique) ou l'acide 2-phényl benzimidazole 5-sulfonique et ses sels, en vue de produire une activité synergique au niveau des indices de protection solaire. Ces systèmes filtrants synergiques imposent l'utilisation d'au moins une phase aqueuse solubilisant le filtre hydrosoluble et d'une phase grasse pour solubiliser le filtre siliconé ; ce qui réduit sensiblement les possibilités de formulation.

Le document WO9737634 décrit des compositions antisolaires à base de diphéhylacrylates, de dérivés de dibenzoylméthane et de benzotriazoles siliciés.

On connaît dans les demandes de brevet EP0967200, DE19746654 DE19755649, EP1008 586, DE 100 07 017, EP 1133980 et EP 1133981 des compositions solaires à base de 4,4-diarylbutadiènes pouvant contenir d'autres filtres complémentaires.

A la suite d'importantes recherches menées dans le domaine de la photoprotection évoqué ci-dessus, la Demanderesse a découvert, de façon inattendue et surprenante, qu'une combinaison de trois familles de composés filtrants particulières et déjà connues en soi dans l'état de l'art -(1) un dérivé de dibenzoylméthane, (2) un dérivé solicié de benzotriazole et (3) un 4,4-diarylbutadiène- permettait, du fait d'un effet de synergie, d'obtenir des compositions antisolaires présentant des facteurs de protection solaire nettement améliorés. Une telle association permettait d'obtenir des compositions solaires dont les indices de protection solaire sont nettement améliorés, et en tous cas largement supérieurs à ceux qui peuvent être obtenus soit avec l'un ou l'autre des filtres utilisé seul.

De plus, l'association particulière de filtres conforme à l'invention peut être facilement incorporée dans une très large gamme de supports cosmétiques.

Cette découverte est à la base de la présente invention.

Ainsi, conformément à l'un des objets de la présente invention, il est maintenant proposé de nouvelles compositions cosmétiques, en particulier antisolaires, qui sont caractérisées par le fait qu'elles comprennent, dans un support cosmétiquement acceptable :
(i) au moins un dérivé silicié à fonction benzotriazole à titre de premier filtre ;
(ii) au moins un dérivé de dibenzoylméthane à titre de deuxième filtre ;
(iii) au moins un composé 4,4-diarylbutadiène à titre de troisième filtre.

La présente invention a également pour objet l'utilisation des compositions ci-dessus pour la fabrication de compositions cosmétiques destinées à la protection de la peau et/ou des cheveux contre le rayonnement ultraviolet, en particulier le rayonnement solaire.

De façon générale lesdits premier, second et troisième filtres sont présents dans les compositions de l'invention dans une proportion produisant une activité synergique au niveau des indices de protection solaire conférés .

D'autres caractéristiques, aspects et avantages de la présente invention apparaîtront à la lecture de la description détaillée qui va suivre.

Par composé 4,4-diarylbutadiène conforme à l'invention, on entend toute molécule comportant au moins un groupe chromophore 4,4-diarylbutadiène. Celle-ci peut se présenter sous forme de composé simple, d'oligomère ou de polymère possédant sur la chaîne des greffons contenant le groupe chromophore.

Les dérivés siliciés à fonction benzotriazole utilisés dans la présente invention sont de préférence des silanes ou des siloxanes à fonction benzotriazole comprenant au moins une unité de formule (1) suivante :

O_{(3-a)/2} Si (R₇)ₐ - G (1)

dans laquelle :
- R₇ représente un radical alkyle en C₁-C₁₀ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante : 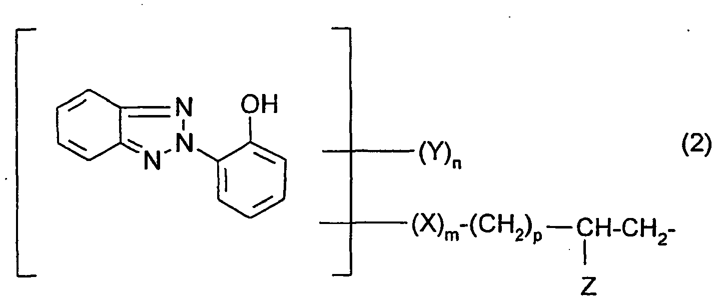
dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement.

Ces composés sont notamment décrits dans les demandes de brevet EP-A-0392883 ; EP-A-0660701 ; EP-A-0708108 ; EP-A-0711778 ; EP-A-711779.

De préférence, les dérivés siliciés utilisés dans le cadre de la présente invention appartiennent à la famille générale des silicones benzotriazoles qui est décrite notamment dans EP-A-0660701 .

Une famille de silicones benzotriazoles convenant particulièrement bien à la réalisation de la présente invention est celle regroupant les composés répondant aux formules (5) ou (6) suivantes : 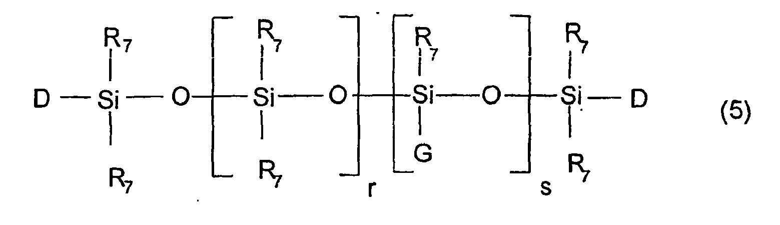 ou 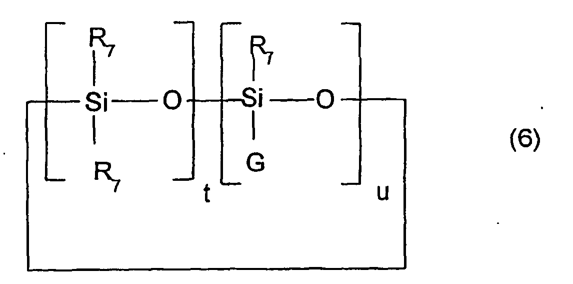 dans lesquelles :
- R₇, identiques ou différents, sont choisis parmi les radicaux alkyles en C₁-C₁₀, phényle, trifluoro-3,3,3 propyle et triméthylsilyloxy, au moins 80% en nombre des radicaux R₇ étant méthyle,
- D, identiques ou différents sont choisis parmi les radicaux R₇ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

Comme cela ressort de la formule (2) donnée ci-dessus, l'accrochage du chaînon -(X)ₘ-(CH₂)ₚ-CH(Z)-CH₂- sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

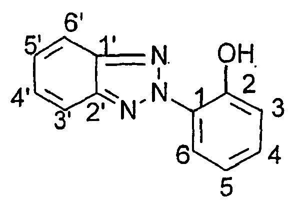

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (5) et (6) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R₇ préférés selon l'invention sont les radicaux méthyle, éthyle, propyle, n-butyle, n-octyle et éthyl-2 hexyle. Encore plus préférentiellement, les radicaux R₇ sont tous des radicaux méthyle.

Parmi les composés de formules (5) ou (6) ci-dessus, on préfère mettre en oeuvre ceux répondant à la formule (5), c'est-à-dire des diorganosiloxanes à chaîne courte linéaire.

Parmi les composés de formules (5) ci-dessus, on préfère mettre en oeuvre ceux pour lesquels les radicaux D sont tous les deux des radicaux R₇

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :
- D est un radical R₇
- R₇ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert.-butyle ou alcoxy en C₁-C₄,
- Z est hydrogène ou méthyle,
- m=0, ou [m=1 et X=O]
- p est égal à 1.

Une famille de silicones benzotriazoles convenant particulièrement bien à l'invention est celle définie par la formule générale (7) suivante : 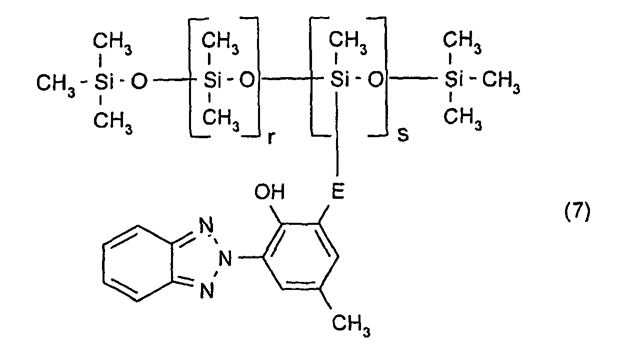 avec 0 ≤ r ≤ 10 ,
1 ≤ s ≤ 10,
et où E représente le radical divalent :

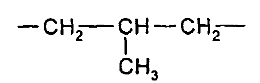

Dans une forme particulièrement préférée de réalisation de l'invention, la silicone benzotriazole est le composé Drométrizole Trisiloxane (nom CTFA) répondant à la formule suivante :

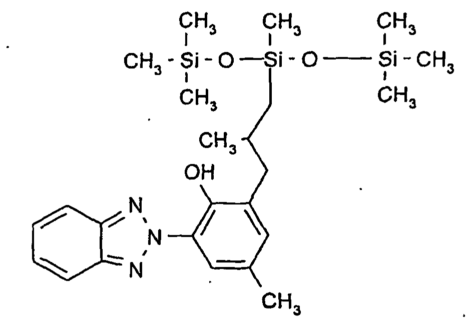

Des procédés convenant à la préparation des produits de formule (1), (5), (6) et (7) ci-dessus sont notamment décrits dans les brevets américains US3,220,972, US3,697,473, US4,340,709, US4,316,033, US4,328,346 et dans les demandes de brevet EP-A-0392883 et EP-A-0742 003.

Le dérivé silicié à fonction benzotriazole peut être présent dans les compositions selon l'invention à des teneurs allant de 0,5 à 15%, de préférence allant de 1 à 10%, en poids, et plus particulièrement de 2 à 8% en poids toujours par rapport au poids total de la composition.

Comme indiqué précédemment, les dérivés du dibenzoylméthane visés par la présente invention sont des produits déjà bien connus en soi et décrits notamment dans les documents FR-A- 2 326 405, FR-A- 2 440 933 et EP-A- 0 114 607, documents dont les enseignements sont, pour ce qui touche à la définition même de ces produits, totalement inclus à titre de références dans la présente description.

Parmi les dérivés du dibenzoylméthane plus particulièrement visés par la présente invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4-tert-butyl-4'-méthoxydibenzoylméthane, notamment celui proposé à la vente sous la dénomination commerciale de "PARSOL 1789" par la Société Hoffmann Laroche, ce filtre répondant à la formule développée (I) suivante :

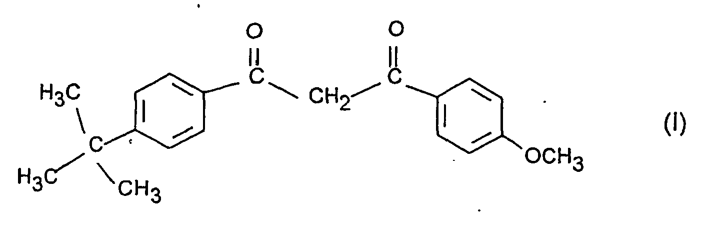

Un autre dérivé du dibenzoylméthane préféré selon la présente invention est le 4-isopropyldibenzoylméthane, filtre vendu sous la dénomination de "EUSOLEX 8020" par la Société Merck, et répondant à la formule développée (II) suivante :

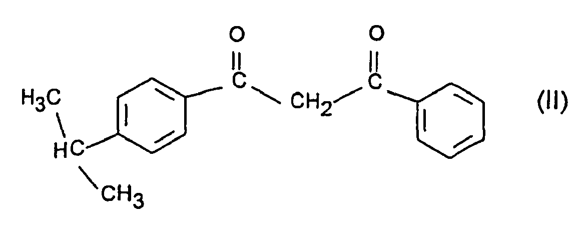

Le ou les dérivés du dibenzoylméthane sont présents dans les compositions conformes à l'invention à des teneurs qui sont de préférence allant de 0,5 à 15% en poids et plus préférentiellement de 1 % à 10 % en poids, et plus particulièrement de 2 à 8% en poids par rapport au poids total de la composition.

Parmi les composés 4,4-diarylbutadiènes conformes à l'invention préférés, on peut choisir les composés répondant à la formule (III) suivante : 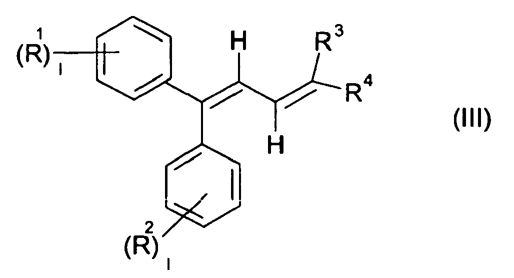 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀, linéaire ou ramifié ; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ linéaire ou ramifié ; un radical monoalkylamino en C₁-C₁₂, linéaire ou ramifié ; un radical dialkylamino en C₁-C₁₂ , linéaire ou ramifié ; un aryle ; un hétéroaryle ou un substituant hydrosolubilisant choisi parmi un groupe carboxylate, un groupe sulfonate ou un reste ammonium ;
- R désigne un groupe COOR⁵ ; COR⁵ ; CONR⁵R⁶ ; CN ; un radical alkyle en C₁₋C_{20,} linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ ; un hétéroaryle en C₃-C₇ ;
- R⁴ désigne un groupe COOR⁶ ; COR⁶ ; CONR⁵R⁶ ; CN ; un radical alkyle en C₁₋C_{20,} linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀; ; un radical bicycloalcényle en C₇-C₁₀ ; un aryle ; un hétéroaryle ;
- R⁵ et R⁶' identiques ou différents, désignent hydrogène; [V]₀-R⁷ , C₁-C₆-alkylène-SO₃U ; C₁-C₆-alkylène-PO₃U ; C₁-C₆-alkylène-N(R⁸)₃⁺A⁻ ; un radical alkyle en C₁-C₂₀, linéaire ou ramifié ; un radical alcényle en C₂-C₁₀ ; un radical cycloalkyle en C₃-C₁₀; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- V désigne un groupe -CH₂-CH₂-W-, -CH₂CH₂CH₂W-, -CH(CH₃)-CH₂-W-, -CH₂-CH₂-CH₂-CH₂-W-, -CH₂-CH(CH₂CH₃)-W- ;
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- W désigne O ou NH ;
- R⁷ et R identiques ou différents, désignent hydrogène, un radical alkyle en C₁-C₆, linéaire ou ramifié ; un radical alcényle en C₂- C₆, linéaire ou ramifié ; un radical acyle en C₁-C₆ linéaire ou ramifié ;
- R⁹ désigne hydrogène, un radical alkyle en C₁-C₆, linéaire ou ramifié ; un radical alcényle en C₂- C₆ ;
- l varie de 1 à 3 ;
- o varie de 0 à 150.

Comme radicaux alkyle , on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple. : éthènyle, n-propènyle, 1-méthyléthènyle, n-butènyle, 1-méthylpropènyle, 2-méthylpropènyle, 1,1-diméthyléthènyle, n-pentènyle, 1-méthylbutènyle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle, 1,1-diméthylbutènyle, 1,2-diméthylbutènyle, 1,3-diméthylbutènyl e,2,2-diméthylbutènyle, 2,3-diméthylbutènyle, 3,3-diméthylbutènyle, 1-éthylbutènyle, 2-éthylbutènyle, 1, 1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en C₁-C₁₂ pour les radicaux R¹ et R², on peut citer : méthoxy, n-propoxy, 1-méthylpropoxy, 1-méthyléthoxy, n-pentoxy, 3-méthylbutoxy, 2,2-diméthylpropoxy, 1-méthyl-1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux cycloalkyles en C₃-C₁₀ pour les radicaux R⁶ et R⁷, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, pour les radicaux R⁶ et R⁷, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres peuvent être saturées par un hydrogène ou un radical alkyle en C₁-C₄.

Comme radicaux acyle, on peut citer par exemple formyle, acétyle, propionyle, ou n-butyryle.

Les groupes bicycloalkyles ou bicycloalcènyles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄₋alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (III) préférentiels sont choisis parmi ceux de formule (IIIa) suivante : 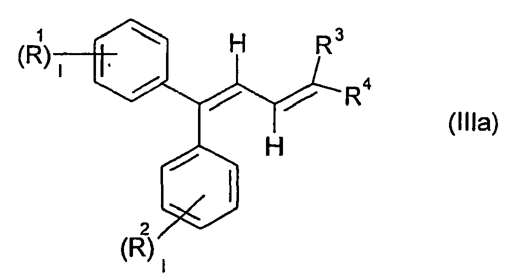 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ; un substituant hydrosolubilisant choisi parmi un groupe carboxylate, un groupe sulfonate ou un reste ammonium ;
- R désigne un groupe COOR⁵ ; CONR⁵R⁶ ; CN ;
- R⁴ désigne un groupe COOR⁶ ; CONR⁵R⁶ ;
- R⁵ désigne hydrogène ; [V]₀-R⁷ ;C₁-C₆-alkylène-SO₃U ;C₁-C₆-alkylène-N(R⁸)₃⁺ A⁻
- R⁶ désigne [V]ₒ-R⁷ ; C₁-C₆-alkylène-SO₃U ; C₁-C₆-alkylène-N(R⁸)₃⁺ A⁻ ;
- V désigne un groupe -CH₂-CH₂-O-, -CH₂CH₂CH₂O-, -CH(CH₃)-CH₂-O-,
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié ;
- l varie de 1 à 3
- o varie de 0 à 50 ;

Les composés de formule (III) encore plus préférentiels sont choisis parmi ceux répondant à la formule (IIIb) suivante : 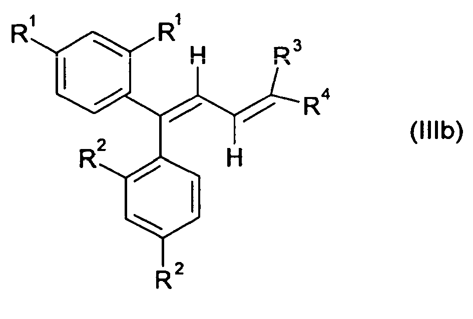 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ;
- R³ désigne un groupe COOR⁵ ; CONR⁵R⁶ ; CN ;
- R⁴ désigne un groupe COOR⁶ ; CONR⁵R⁶ ;
- R⁵ désigne hydrogène ; [V]ₒ-R⁷ ;C₁-C₆-alkylène-SO₃Y ;C₁-C₆-alkylène-N(R⁸)₃⁺A⁻ ;
- R⁶ désigne [V]ₒ-R⁷ ; C₁-C₆-alkylene-SO₃U ; C₁-C₆-alkylène-N(R⁸)₃⁺ A⁻ ;
- V désigne un groupe -CH₂-CH₂-O-, -CH₂CH₂CH₂O-, -CH(CH₃)-CH₂-O-,
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié ;
- o varie de 0 à 50.

Les composés de formule (III) encore plus préférentiels sont choisis parmi ceux répondant à la formule (IIIc) suivante : 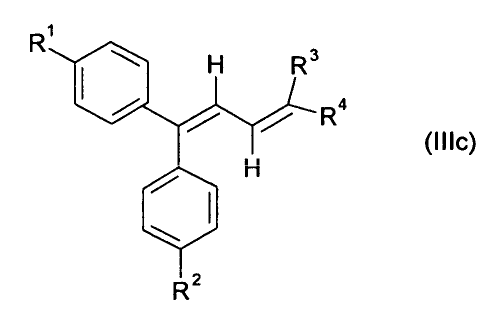 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ;
- R³ désigne un groupe COOR⁵ ; CONR⁵R⁶ ; CN ;
- R⁴ désigne un groupe COOR⁶ ; CONR⁵R⁶;
- R⁵ désigne hydrogène ; [V]ₒ-R⁷ ;C₁-C₆-alkylène-SO₃U ; C₁-C₆-alkylène-N(R⁸)₃⁺A⁻
- R⁶ désigne [V]ₒ-R⁷ ; C₁-C₆-alkylène-SO₃U ; C₁-C₆-alkylène-N(R⁸)₃⁺A⁻ ;
- V désigne un groupe -CH₂-CH₂-O-, -CH₂CH₂CH₂O-, -CH(CH₃)-CH₂-O-,
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷ , R⁸ et R⁹ identiques ou différents, désignent hydrogène, un radical alkyle en C₁-C₃, linéaire ou ramifié ;
- o varie de 0 à 50.

Les composés de formule (III) encore plus particulièrement préférés sont choisis parmi les composés suivants :

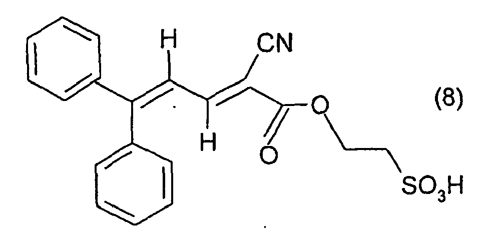

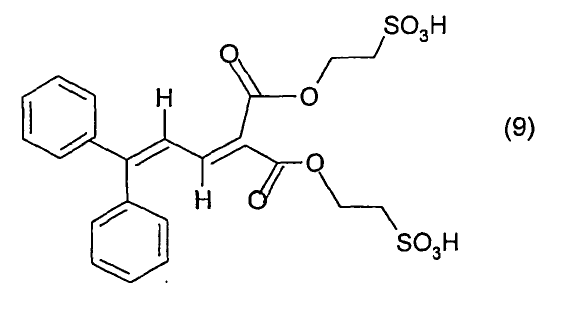

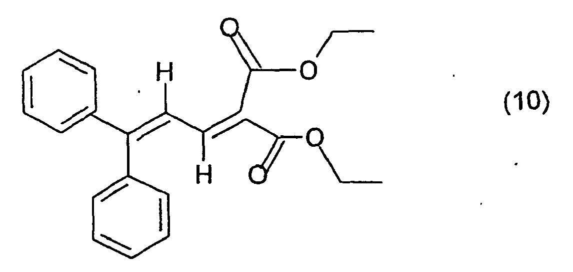

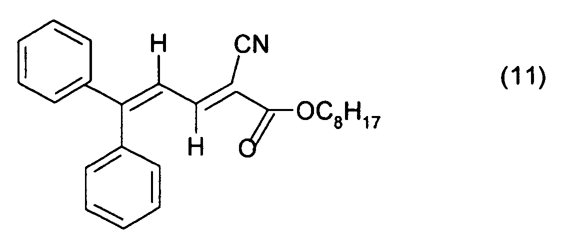

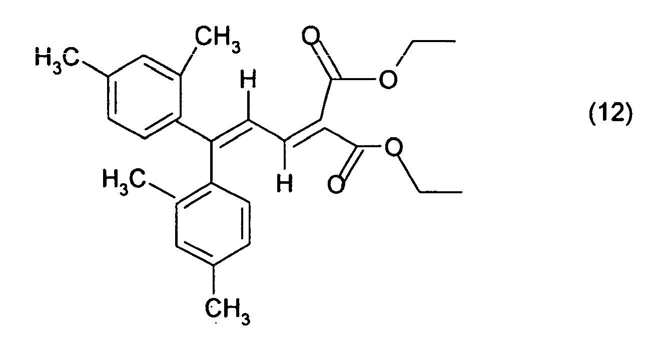

Les composés de formule (III) tels que définis ci-dessus sont connus en eux-mêmes et leurs structures et leurs synthèses sont décrites dans les demandes de brevet EP0967200, DE19746654 et DE19755649 (faisant partie intégrante du contenu de la description).

Parmi les composés 4,4-diarylbutadiènes conformes à l'invention préférés, on peut également citer les oligomères répondant à la formule (IV) suivante : 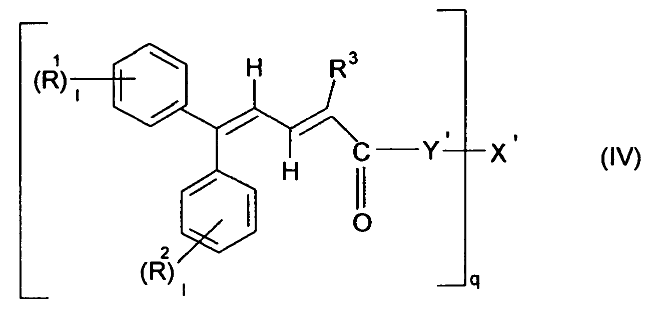 dans laquelle le système diène est de configuration Z,Z ; Z,E ; E,Z ou E,E ou des mélanges desdites configurations et où :
- R¹, R², R³ et I ont les mêmes significations indiquées dans la formule (III) précédente ;
- Y' désigne un groupe -O- ou -NR¹⁰-
- R¹⁰ désigne hydrogène ; un radical alkyle en C₁-C₂₀, linéaire ou ramifié ; un radical alcényle en C₂- C₁₀; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- I varie de 2 à 10.

X' est un reste polyol contenant de 2 à 10 groupes hydroxyles et notamment :

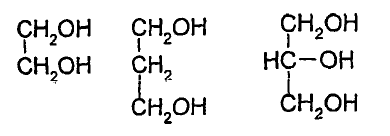

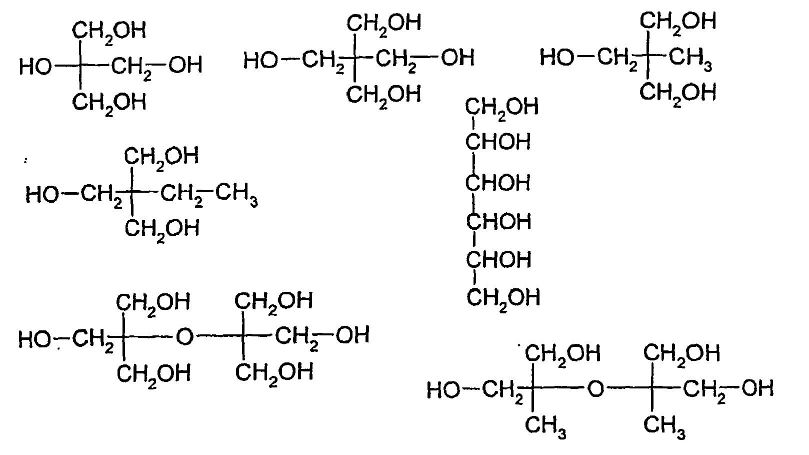

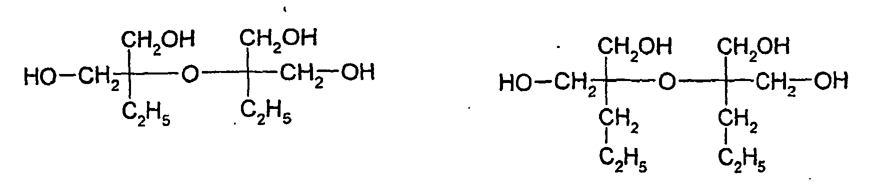

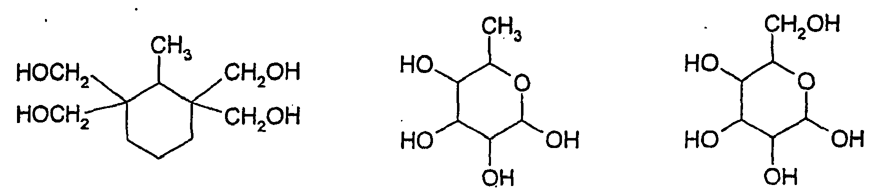

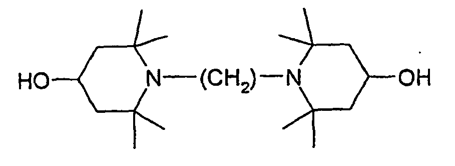

Les composés plus préférentiels de formule (IV) sont ceux pour lesquels :
- R¹ et R², identiques ou différents, désignent hydrogène, un radical alkyle en C₁-C₁₂ ; un radical alcoxy en C₁-C₈ ; un substituant hydrosolubilisant choisi parmi un groupe carboxylate, un groupe sulfonate ou un reste ammonium ;
- R désigne un groupe COOR⁵; CONR⁵R⁶; CN ; un radical cycloalkyle en C₃₋C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀ ; naphtyle ou phényle éventuellement substitué ;
- X' désigne un reste de polyol comprenant de 2 à 6 groupes hydroxy et plus particulièrement de 2 à 4.

Les composés encore plus préférentiels de formule (IV) sont ceux pour lesquels : - X' désigne un reste d'éthanol ou de pentaérythrol.

Les composés de formule (IV) encore plus particulièrement préférés sont choisis parmi les composés suivants :

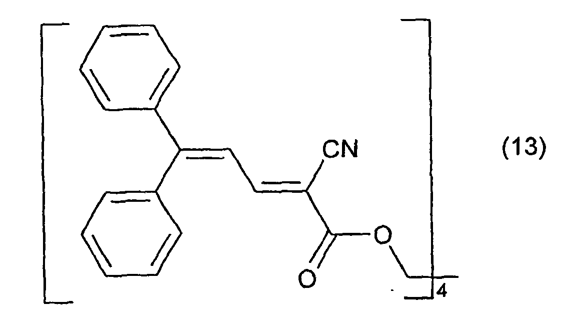

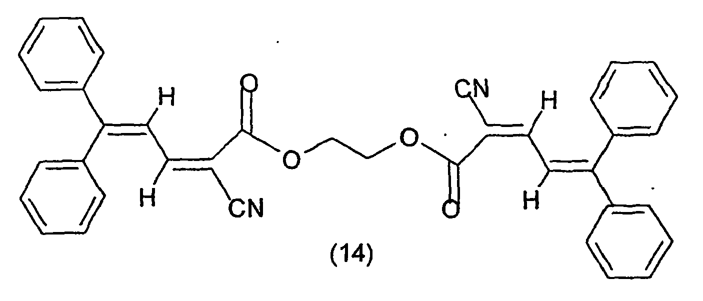

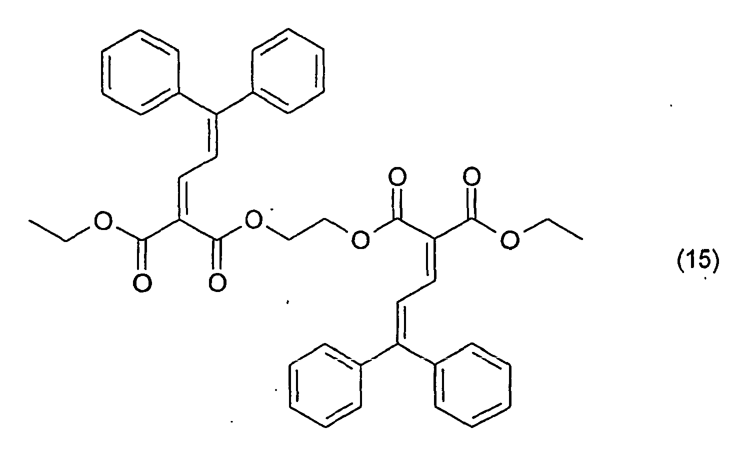

Les composés de formule (IV) tels que définis ci-dessus sont connus en eux-mêmes et leurs structures et leurs synthèses sont décrites dans la demande de brevet EP-A-1 008586 (faisant partie intégrante du contenu de la description). ,

Les composés 4,4-diarylbutadiène conformes à l'invention sont généralement présents dans la composition de l'invention à des teneurs qui de préférence varient de 0,5 à 15% en poids et plus préférentiellement de 1 % à 10 % en poids, et plus particulièrement de 2 à 8% en poids par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés cinnamiques ; les dérivés salicyliques, les dérivés du camphre autres que le p-méthyl-benzylidène camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β,β'-diphénylacrylate, les dérivés de benzotriazole ; les dérivés de benzalmalonate ; les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres autres que ceux de l'invention tels que ceux décrits notamment dans la demande WO-93/04665 ; les dimères dérivés d'α-alkylstyrène tels que ceux décrits dans la demande de brevet DE19855649.

Comme exemples de filtres organiques complémentaires actifs dans l'UV-A et/ou l'UV-B, on peut citer désignés ci-dessus sous leur nom INCI :

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- -Diisopropyl Methylcinnamate,
- Glyceryl Ethylhexanoate Dimethoxycinnamate

### Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

### Dérivé du benzylidène camphre :

- 3-Benzylidene camphor fabriqué sous le nom « MEXORYL SD» par CHIMEX,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MESORYL SW » par CHIMEX,

### Dérivés de benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

### Dérivés de triazine :

- Anisotriazine vendu sous le nom commercial «TINOSORB S » par CIBA SPECIALTY CHEMICALS
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

### Dérivés du phenyl benzotriazole :

- Drometrizole Trisiloxane vendu sous le nom « SILATRIZOLE » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

- Menthyl anthranilate vendu sous le nom commercial commercial « NEO HELIOPAN MA » par HAARMANN et REIMER,

### Dérivés d'imidazolines :

- Ethylhexyl Dimethoxybenzylidene Dioxoimidazoline Propionate,

### Dérivés de benzalmalonate :

- Polyorganosiloxane à fonction benzalmalonate vendu sous la dénomination commerciale « PARSOL SLX » par HOFFMANN LA ROCHE et leurs mélanges.

Les filtres UV organiques solubles plus particulièrement préférés sont choisis parmi les composés suivants :
- Ethylhexyl Salicylate,
- Ethylhexyl Methoxycinnamate,
- Octocrylene,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s- triazine.
- Methylène bis-Benzotriazolyl Tetramethylbutylphénol et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Bien entendu, l'homme de l'art veillera à choisir ce ou ces éventuels composés complémentaires et/ou leurs quantités de manière telle que les propriétés avantageuses, en particulier la synergie des facteurs de protection, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en' particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un objet de l'invention est l'utilisation d'une composition telle que définie précédemment pour la fabrication d'une composition cosmétique ou dermatologique destinées à la protection de la peau et/ou des cheveux contre le rayonnement ultraviolet, en particulier le rayonnement solaire.

Des exemples concrets, mais nullement limitatifs, illustrant l'invention, vont maintenant être donnés.

| **COMPOSITION** | **EX 1** |
|---|---|
| Mélange mono /distéarate de glycerol / stéarate de polyéthylène | 2 |
| glycol (100 OE) (ARLACEL 165 FL - ICI) | |
| Alcool stéarylique (LANETTE 18 - HENKEL) | 1 |
| Acide stéarique d'huile de palme | 2.5 |
| (STEARINE TP - STEARINERIE DUBOIS) | |
| Poly diméthylsiloxane | 0.5 |
| (DOW CORNING 200 FLUID - DOW CORNING) | |
| Benzoate d'alcools en C12/C15 | 20 |
| (WITCONOL TN -WITCO) | |
| Triéthanolamine | 0.5 |
| Butyl Methoxydibenzoylmethane | 2 |
| (PARSOL 1789 HOFFMANN LA ROCHE) | |
| Drometrizole Trisiloxane | 5 |
| (SILATRIZOLE , RHODIA CHIMIE) | |
| Composé de formule (8) | 8 |
| Glycérine | 4 |
| Triéthanolamine | 0.3 |
| Acide polyacrylique | 0.4 |
| (SYNTHALEN K - 3V) | |
| Conservateurs | qs |
| Eau démineralisée qsp | 100 g |

| **COMPOSITION** | **EX 2** |
|---|---|
| Mélange d'alcool cétylstéarylique et d'alcool cétylstéarylique | 7 |
| oxyéthyléné (33 OE) 80/20 (SINNOWAX AO -HENKEL) | |
| Mélange de mono et distéarate de glycérol | 2 |
| (CERASYNT SD-V ISP) | |
| Alcool cétylique | 1.5 |
| Polydiméthyl siloxane | 1.5 |
| (DOW CORNING 200 FLUID -DOW CORNING) | |
| Huile de vaseline | 15 |
| Butyl Methoxydibenzoylmethane | 3 |
| (PARSOL 1789 HOFFMANN LA ROCHE) | |
| Composé de formule (10) | 6 |
| Drometrizole Trisiloxane | 3 |
| (SILATRIZOLE , RHODIA CHIMIE) | |
| Glycérine | 15 |
| Conservateurs | qs |
| Eau déminéralisée qsp | 100 g |

| **COMPOSITION** | **EX 3** |
|---|---|
| Mélange mono /distéarate de glycerol / stéarate de polyéthylène | 2 |
| glycol (100 OE) (ARLACEL 165 FL - ICI) | |
| Alcool stéarylique (LANETTE 18 - HENKEL) | 1 |
| Acide stéarique d'huile de palme | 2.5 |
| (STEARINE TP - STEARINERIE DUBOIS) | |
| Polydiméthylsiloxane | 0.5 |
| (DOW CORNING 200 FLUID - DOW CORNING) | |
| Benzoate d'alcools en C12/C15 | 20 |
| (WITCONOL TN -WITCO) | |
| Triéthanolamine | 0.5 |
| Butyl Methoxydibenzoylmethane | 2 |
| (PARSOL 1789 HOFFMANN LA ROCHE) | |
| Drometrizole Trisiloxane | 6 |
| (SILATRIZOLE , RHODIA CHIMIE) | |
| Composé de formule (11) | 8 |
| Oxybenzone, | 10 |
| (UVINUL M40, BASF) | |
| Glycérine | 4 |
| Triéthanolamine | 0.3 |
| Acide polyacrylique | 0.4 |
| (SYNTHALEN K - 3V) | |
| Conservateurs | qs |
| Eau démineralisée qsp | 100 g |

| **COMPOSITION** | **EX 4** |
|---|---|
| Mélange d'alcool cétylstéarylique et d'alcool cétylstéarylique | 7 |
| oxyéthyléné (33 OE) 80/20 (SINNOWAX AO -HENKEL) | |
| Mélange de mono et distéarate de glycérol | 2 |
| (CERASYNT SD-V ISP) | |
| Alcool cétylique | 1.5 |
| Polydiméthyl siloxane | 1.5 |
| (DOW CORNING 200 FLUID -DOW CORNING) | |
| Huile de vaseline | 15 |
| Butyl Methoxydibenzoylmethane | 2 |
| (PARSOL 1789 HOFFMANN LA ROCHE) | |
| Composé de formule (13) | 6 |
| Drometrizole Trisiloxane | 6 |
| (SlLATRIZOLE , RHODIA CHIMIE) | |
| Glycérine | 15 |
| Conservateurs | qs |
| Eau déminéralisée qsp | 100 g |

| **COMPOSITION** | **EX 5** |
|---|---|
| Mélange mono /distéarate de glycerol / stéarate de polyéthylène | 2 |
| glycol (100 OE) | |
| (ARLACEL 165 FL - ICI) | |
| Alcool stéarylique | 1 |
| (LANETTE 18 - HENKEL) | |
| Acide stéarique d'huile de palme | 2.5 |
| (STEARINE TP - STEARINERIE DUBOIS) | |
| Poly diméthylsiloxane | 0.5 |
| (DOW CORNING 200 FLUID - DOW CORNING) | |
| Benzoate d'alcools en C12/C15 | 20 |
| (WITCONOL TN -WITCO) | |
| Triéthanolamine | 0.5 |
| Butyl Methoxydibenzoylmethane | 3 |
| (PARSOL 1789 HOFFMANN LA ROCHE) | |
| Drometrizole Trisiloxane | 5 |
| (SILATRIZOLE , RHODIA CHIMIE) | |
| Composé de formule (15) | 5 |
| Glycérine | . 4 |
| Triéthanolamine | 0.3 |
| Acide polyacrylique | 0.4 |
| (SYNTHALEN K - 3V) | |
| Conservateurs | qs |
| Eau démineralisée qsp | 100 g |

## Revendications

1. Composition cosmétique, en particulier antisolaires, **caractérisée par le fait qu'**elle comprend, dans un support cosmétiquement acceptable :
(i) au moins un dérivé silicié à fonction benzotriazole à titre de premier filtre ;
(ii) au moins un dérivé du dibenzoylméthane à titre de deuxième filtre ;
(iii) au moins un composé 4,4-diarylbutadiène à titre de troisième filtre.

2. Composition selon la revendication 1, où lesdits premier, second et troisième filtres sont présents dans ladite composition dans une proportion produisant une activité synergique au niveau des indices de protection solaire conférés .

3. Composition selon la revendication 1 ou 2, où le dérivé silicié à fonction benzotriazole est choisi parmi les silanes et/ou les polyorganosiloxanes à fonction benzotriazole comprenant au moins une unité de formule (1) suivante :
O_{(3-a)/2} Si (R₇)ₐ - G (1)
dans laquelle :
- R₇ représente un radical alkyle en C₁-C₁₀ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante : 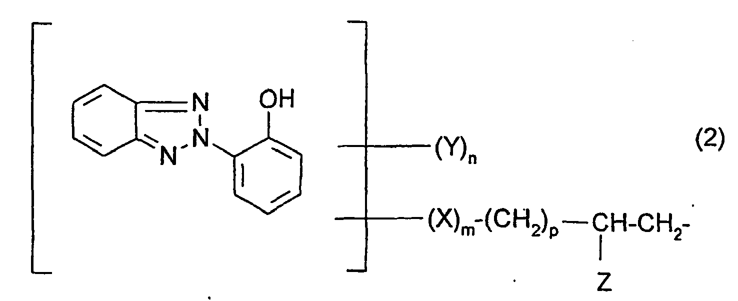
dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement.

4. Composition selon l'une quelconque des revendications 1 à 3, **caractérisée par le fait que** le dérivé silicié à fonction benzotriazole répond à l'une des formules (5) ou (6) suivantes : 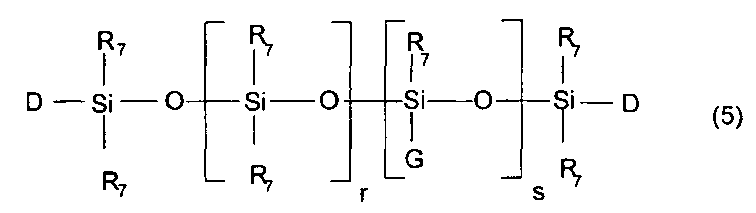 ou 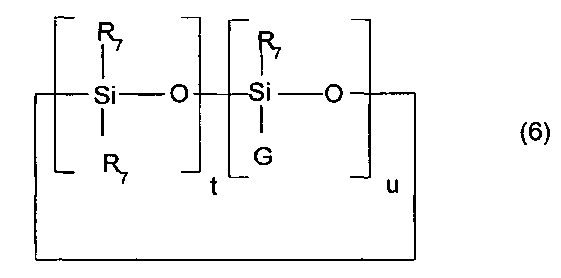 dans lesquelles :
- R₇, identiques ou différents, sont choisis parmi les radicaux alkyles en C₁-C₁₀, phényle, trifluoro-3,3,3 propyle et triméthylsilyloxy, au moins 80% en nombre des radicaux R₇ étant méthyle,
- D, identiques ou différents sont choisis parmi les radicaux R₇ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) définie dans la revendication 2.

5. Composition selon la revendication 4, **caractérisée par le fait que** dérivé silicié à fonction benzotriazole répond à la formule (7) suivante : 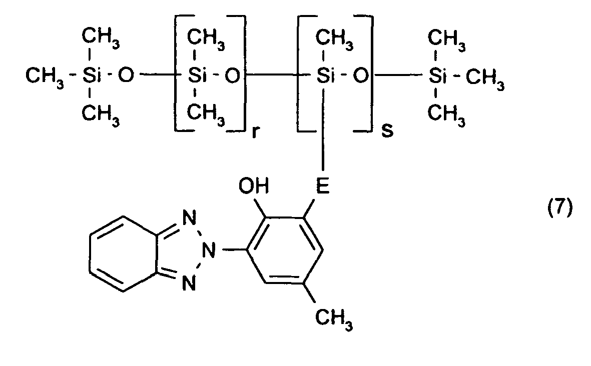 avec 0 ≤ r ≤ 10,
1 ≤ s ≤ 10,
et où E représente le radical divalent :

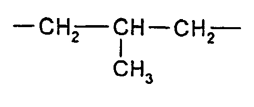

6. Composition selon la revendication 5, **caractérisée par le fait que** le dérivé silicié à fonction benzotriazole est le Drométrizole Trisiloxane de formule suivante :

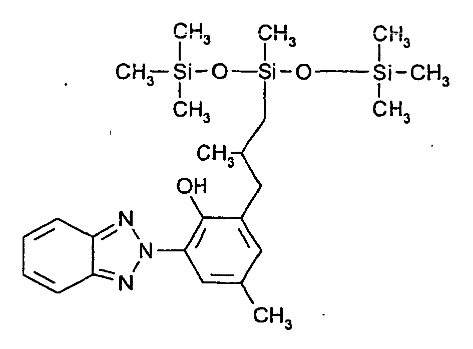

7. Composition selon l'une quelconque des revendications 1 à 6, **caractérisée par le fait que** les dérivés siliciés à fonction benzotriazole sont présents à une teneur allant de 0,5% à 15 %, de préférence de 1 % à 10 % en poids, par rapport au poids total de la composition.

8. Composition selon l'une quelconque des revendications 1 à 7, **caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

9. Composition selon la revendication 8, **caractérisée par le fait que** le dérivé de dibenzoylméthane est le 4-(ter.-butyl) 4'-méthoxy dibenzoylméthane.

10. Composition selon la revendication 8, **caractérisée par le fait que** le dérivé de dibenzoylméthane est le 4-isopropyl-dibenzoylméthane.

11. Composition selon l'une quelconque des revendications 1 à 10, où le dérivé du dibenzoylméthane est présent à des teneurs allant de 0,5 à 15% en poids et de préférence de 1 % à 10 % en poids, et plus particulièrement de 2 à 8% en poids par rapport au poids total de la composition.

12. Composition l'une quelconque des revendications 1 à 11, où le composé 4,4-diarylbutadiène répond à la formule (III) suivante : 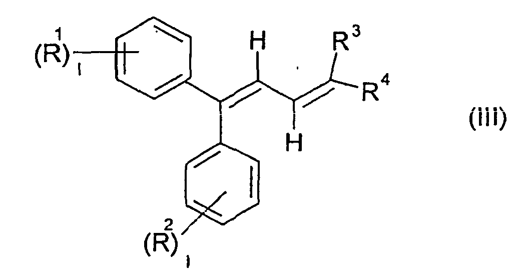 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀, linéaire ou ramifié ; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ linéaire ou ramifié ; un radical monoalkylamino en C₁-C₁₂, linéaire ou ramifié ; un radical dialkylamino en C₁-C₁₂ , linéaire ou ramifié ; un aryle ; un hétéroaryle ou un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ; CN ; un radical alkyle en C₁₋C_{20,} linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ ; un hétéroaryle en C₃-C₇ ;
- R⁴ désigne un groupe COOR⁶ ; COR⁶ ; CONR⁵R⁶ ; CN ; un radical alkyle en Ci-C_{20,} linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle ;
- R⁵ et R⁶' identiques ou différents, désignent hydrogène ; [V]₀-R⁷ , C₁-C₆-alkylène-SO₃U ; C₁-C₆-alkylène-PO₃U ; C₁-C₆-alkylène-N(R⁸)₃⁺A⁻ ; un radical alkyle en C₁-C₂₀, linéaire ou ramifié ; un radical alcényle en C₂-C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- V désigne un groupe -CH₂-CH₂-W-, -CH₂CH₂CH₂W-, -CH(CH₃)-CH₂-W-,
- CH₂-CH₂-CH₂-CH₂-W-, -CH₂-CH(CH₂CH₃)-W- ;
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- W désigne O ou NH ;
- R⁷ et R identiques ou différents, désignent hydrogène, un radical alkyle en C₁-C₆, linéaire ou ramifié ; un radical alcényle en C₂- C₆, linéaire ou ramifié ; un radical acyle en C₁-C₆ linéaire ou ramifié ;
- R⁹ désigne hydrogène, un radical alkyle en C₁-C₆, linéaire ou ramifié ; un radical alcényle en C₂- C₆ ;
- l varie de 1 à 3 ;
- o varie de 1 à 150.

13. Composition selon la revendication 12, où le composé de formule (III) est choisi parmi ceux de formule (IIIa) suivante : 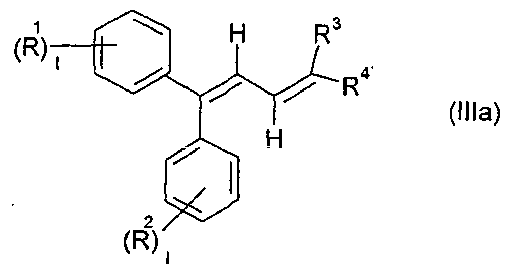 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈; un radical alcoxy en C₁-C₈ ; un substituant hydrosolubilisant choisi parmi un groupe carboxylate, un groupe sulfonate ou un reste ammonium ;
- R désigne un groupe COOR⁵ ; CONR⁵R⁶; CN ;
- R⁴ désigne un groupe COOR⁶ ; CONR⁵R⁶ ;
- R⁵ désigne hydrogène ; [V]ₒ-R⁷ ;C₁-C₆-alkylène-SO₃U ;C₁-C₆-alkylène-N(R⁸)₃⁺A⁻
- R⁶ désigne [V]ₒ-R⁷ ; C₁-C₆-alkylène-SO₃U ; C₁-C₆-alkylène-N(R⁸)₃⁺A⁻ ;
- V désigne un groupe -CH₂-CH₂-O-, -CH₂CH₂CH₂O-, -CH(CH₃)-CH₂-O-,
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷ , R⁸ et R⁹ identiques ou différents, désignent hydrogène, un radical alkyle en C₁-C₃, linéaire ou ramifié ;
- I varie de 1 à 3
- o varie de 0 à 50.

14. Composition selon la revendication 13, où le composé de formule (III) est choisi parmi ceux de formule formule (IIIb) suivante : 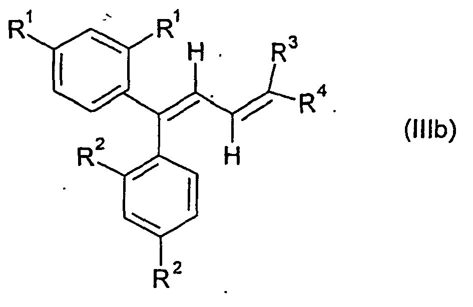 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ;
- R³ désigne un groupe COOR⁵ ; CONR⁵R⁶ ; CN ;
- R⁴ désigne un groupe COOR⁶ ; CONR⁵R⁶ ;
- R⁵ désigne hydrogène ; [V]ₒ-R⁷ ;C₁-C₆-alkylène-SO₃U ; C₁-C₆-alkylène-N(R⁸)₃⁺A⁻
- R⁶ désigne [V]ₒ-R⁷ ; C₁-C₆-alkylène-SO₃U ; C₁-C₆-alkylène-N(R⁸)₃⁺ A⁻ ;
- V désigne un groupe -CH₂-CH₂-O-, -CH₂CH₂CH₂O-, -CH(CH₃)-CH₂-O-,
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷ , R⁸ et R⁹ identiques ou différents, désignent hydrogène, un radical alkyle en C₁-C₃, linéaire ou ramifié ;
- o varie de 0 à 50.

15. Composition selon la revendication 14, où le composé de formule (III) est choisi parmi ceux répondant à la formule (IIIc) suivante : 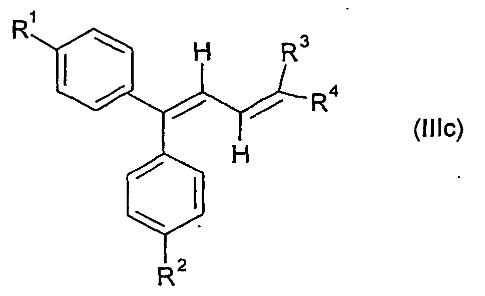 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ;
- R³ désigne un groupe COOR⁵ ; CONR⁵R⁶ ; CN ;
- R⁴ désigne un groupe COOR⁶ ; CONR⁵R⁶ ;
- R⁵ désigne hydrogène ; [V]ₒ-R⁷ ;C₁-C₆-alkylène-SO₃U ;C₁-C₆-alkylène-N(R⁸)₃⁺A⁻
- R⁶ désigne [V]ₒ-R⁷ ; C₁-C₆-alkylène-SO₃U ; C₁-C₆-alkylène-N(R⁸)₃⁺A⁻ ;
- V désigne un groupe -CH₂-CH₂-O-, -CH₂CH₂CH₂O-, -CH(CH₃)-CH₂-O-,
- A désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié ;
- o varie de 0 à 50,

16. Composition selon la revendication 12, où le composé 4,4-diarylbutadiène est choisi parmi les composés suivants :

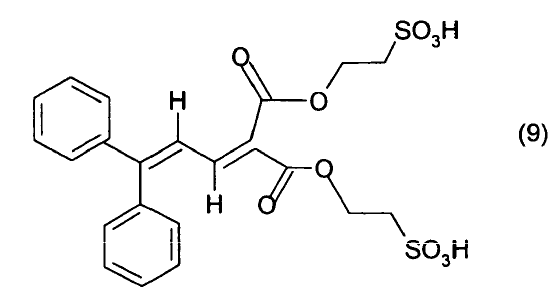

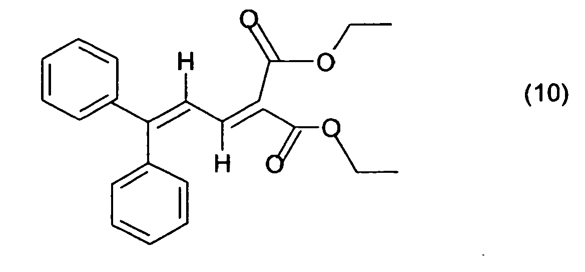

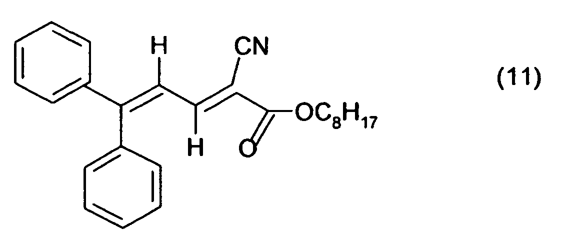

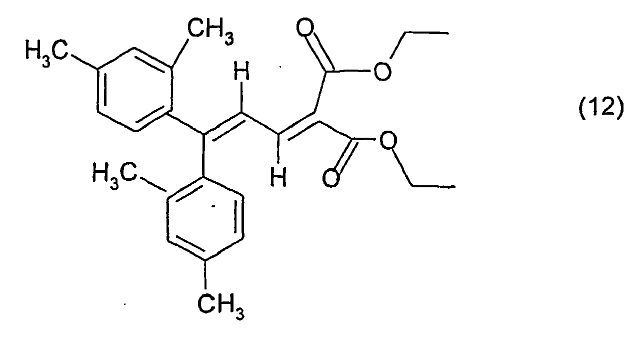

17. Composition selon l'une quelconque des revendications 1 à 11, où le composé 4,4-diarylbutadiène est un oligomère répondant à la formule (IV) suivante : 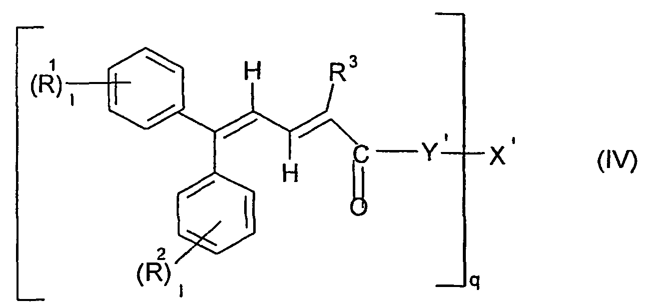 dans laquelle le système diène est de configuration Z,Z ; Z,E ; E,Z ou E,E ou des mélanges desdites configurations et où :
- R¹, R² R³ et I ont les mêmes significations indiquées dans la formule (III) dans la revendication 11 ;
- Y' désigne un groupe ―O- ou ―NR¹⁰-
- R¹⁰ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à 10 groupes hydroxy et de valence q ; la chaîne carbonée dudit reste pouvant être interrompue par un ou plusieurs atomes de souffre ou d'oxygène ; un ou plusieurs groupes imines ou un ou plusieurs alkylimino en C₁₋C₄ ;
- q varie de 2 à 10.

18. Composition selon la revendication 17, où le composé de formule (IV) est choisi parmi ceux pour lesquels :
- R¹ et R², identiques ou différents, désignent hydrogène, un radical alkyle en C₁-C₁₂ ; un radical alcoxy en C₁-C₈ ; un substituant hydrosolubilisant choisi parmi un groupe carboxylate, un groupe sulfonate ou un reste ammonium ;
- R désigne un groupe COOR⁵ ; CONR⁵R⁶; CN ; un radical cycloalkyle en C₃₋C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀ ; naphtyle ou phényle éventuellement substitué ;
- X' désigne un reste de polyol comprenant de 2 à 6 groupes hydroxy et plus particulièrement de 2 à 4.

19. Composition selon la revendication 18, où le composé de formule (IV) est choisi parmi ceux pour lesquels :
- X' désigne un reste d'éthanol ou de pentaérythrol.

20. Composition selon la revendication 19, où le composé de formule (IV) est choisi parmi les composés suivants :

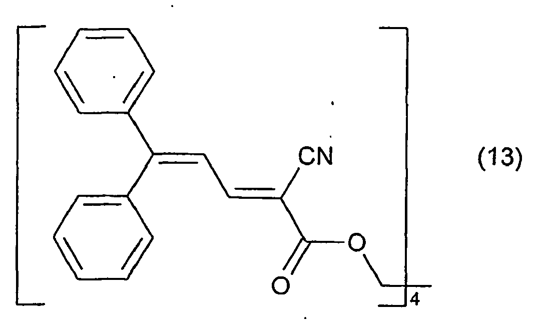

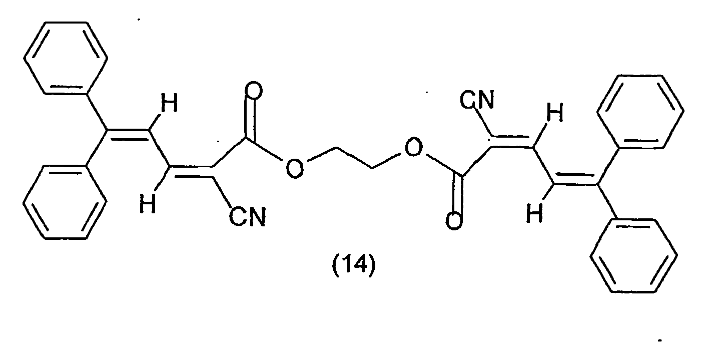

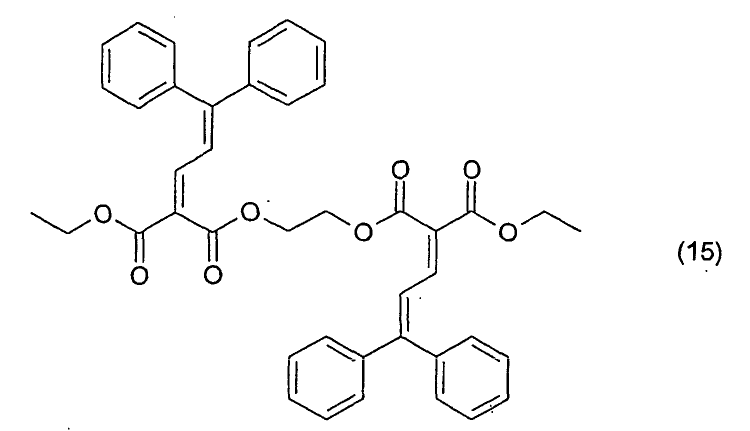

21. Composition selon l'une quelconque des revendications 1 à 20, **caractérisée par le fait que** le composé 4,4-diarylbutadiène est présent à des teneurs allant de 0,5 à 15% en poids et de préférence de 1 % à 10 % en poids, et plus particulièrement de 2 à 8% en poids par rapport au poids total de la composition.

22. Composition selon l'une quelconque des revendications 1 à 21, **caractérisée par le fait qu'**elle contient en plus d'autres filtres organiques actifs dans l'UV-A et/ou l'UV-B.

23. Composition selon la revendication 22, où le ou les filtres UV organiques complémentaires sont choisis parmi les anthranilates ; les dérivés cinnamiques ; les dérivés salicyliques, les dérivés du camphre autres que le p-méthyl benzylidène camphre ; les dérivés de triazine ; les dérivés de la benzophénone ; les dérivés de β,β'-diphénylacrylate, les dérivés de benzotriazole ; les dérivés de benzalmalonatye ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres autres que les dérivés siliciés de benzotriazole ; les dimères dérivés d'α-alkylstyrène.

24. Composition selon la revendication 23, **caractérisée par le fait que** le ou les filtres UV organiques sont choisis parmi les composés suivants :
- Ethylhexyl Salicylate,
- Ethylhexyl Methoxycinnamate,
- Octocrylene,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s- triazine.
- Methylène bis-Benzotriazolyl Tetramethylbutylphénol
et leurs mélanges.

25. Composition selon l'une quelconque des revendications 1 à 24, **caractérisée par le fait qu'**elle comprend en outre, des pigments ou des nanopigments d'oxydes métalliques, enrobés ou non.

26. Composition selon la revendication 25, **caractérisée par le fait que** lesdits pigments ou nanopigments sont choisis parmi les oxydes de titane, de zinc, de fer, de zirconium, de cérium et leurs mélanges, enrobés ou non.

27. Composition selon l'une quelconque des revendications 1 à 26, **caractérisée par le fait qu'**elle comprend en outre au moins un agent de bronzage et/ou de brunissage artificiel de la peau.

28. Composition selon l'une quelconque des revendications 1 à 27, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

29. Composition selon l'une quelconque des revendications 1 à 28, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

30. Composition selon l'une quelconque des revendications 1 à 29, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

31. Composition selon l'une quelconque des revendications 1 à 29, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

32. Utilisation d'une composition telle que définie dans les revendications 1 à 29 pour la fabrication de compositions dermatologiques destinées à la protection de la peau et/ou des cheveux contre le rayonnement ultraviolet, en particulier le rayonnement solaire.

33. Utilisation d'un composé 4,4-diarylbutadiène tel que défini dans les revendications précédentes pour la fabrication de compositions dermatologiques destinées à la protection de la peau et/ou des cheveux contre le rayonnement ultraviolet, en particulier le rayonnement solaire comprenant au moins un filtre du type dérivé silicié de benzotriazole et un filtre du type dérivé de dibenzoylméthane dans le but de produire un effet synergique au niveau des indices de protection solaire conférés.

34. Utilisation d'une composition cosmétique telle que définie dans les revendications 1 à 29 pour la protection de la peau et/ou des cheveux contre le rayonnement ultraviolet, en particulier le rayonnement solaire.

35. Utilisation d'une composition cosmétique comprenant un composé 4,4-diarylbutadiène pour la protection de la peau et/ou des cheveux contre le rayonnement ultraviolet, en particulier le rayonnement solaire comprenant au moins un filtre du type dérivé silicié de benzotriazole et un filtre du type dérivé de dibenzoylméthane dans le but de produire un effet synergique au niveau des indices de protection solaire conférés.

## Claims

1. Cosmetic composition, in particular an antisun composition, **characterized in that** it comprises, in a cosmetically acceptable vehicle:
(i) at least one silicon derivative with a benzotriazole functional group, as first screening agent;
(ii) at least one dibenzoylmethane derivative, as second screening agent;
(iii) at least one 4,4-diarylbutadiene compound, as third screening agent.

2. Composition according to Claim 1, where the said first, second and third screening agents are present in the said composition in a proportion producing a synergistic activity with respect to the sun protection factors conferred.

3. Composition according to Claim 1 or 2, where the silicon derivative with a benzotriazole functional group is chosen from silanes and/or polyorganosiloxanes with a benzotriazole functional group comprising at least one unit of following formula (1):
O_{(3-a)/2}Si(R₇)ₐ-G (1)
in which:
- R₇ represents an optionally halogenated C₁-C₁₀ alkyl radical or a phenyl radical or a trimethylsilyloxy radical,
- a is an integer chosen between 0 and 3 inclusive,
- and the G symbol denotes a monovalent radical bonded directly to a silicon atom which corresponds to the following formula (2): 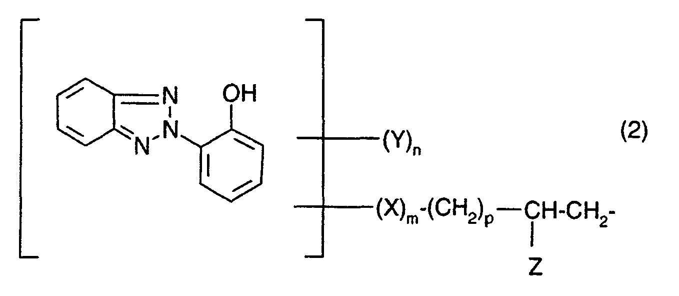
in which:
- Y, which are identical or different, are chosen from C₁-C₈ alkyl radicals, halogens and C₁-C₄ alkoxy radicals, it being understood that, in the latter case, two adjacent Y radicals on the same aromatic nucleus can together form an alkylidenedioxy group in which the alkylidene group comprises from 1 to 2 carbon atoms,
- X represents O or NH,
- Z represents hydrogen or a C₁-C₄ alkyl radical,
- n is an integer between 0 and 3 inclusive,
- m is 0 or 1,
- p represents an integer between 1 and 10 inclusive.

4. Composition according to any one of Claims 1 to 3, **characterized in that** the silicon derivative with a benzotriazole functional group corresponds to one of the following formulae (5) and (6): 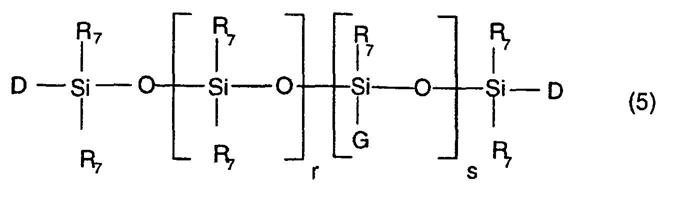 or 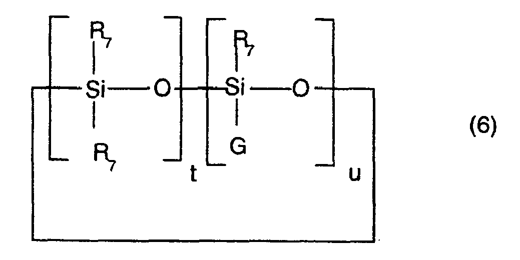 in which
- R₇, which are identical or different, are chosen from C₁-C₁₀ alkyl, phenyl, 3,3,3-trifluoropropyl and trimethylsilyloxy radicals, at least 80% by number of the R₇ radicals being methyl,
- D, which are identical or different, are chosen from R₇ radicals and the G radical,
- r is an integer between 0 and 50 inclusive and s is an integer between 0 and 20 inclusive and, if s = 0, at least one of the two D symbols denotes G,
- u is an integer between 1 and 6 inclusive and t is an integer between 0 and 10 inclusive, it being understood that t + u is equal to or greater than 3,
- and the G symbol corresponds to the formula (2) defined in Claim 2.

5. Composition according to Claim 4, **characterized in that** the silicon derivative with a benzotriazole functional group corresponds to the following formula (7): 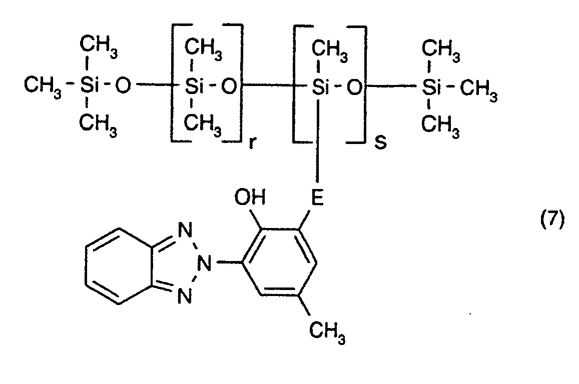 with 0 ≤ r ≤ 10,
1 ≤ s ≤ 10,
and where E represents the divalent radical:

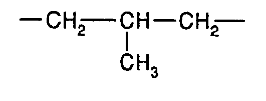

6. Composition according to Claim 5, **characterized in that** the silicon derivative with a benzotriazole functional group is Drometrizole Trisiloxane of following formula:

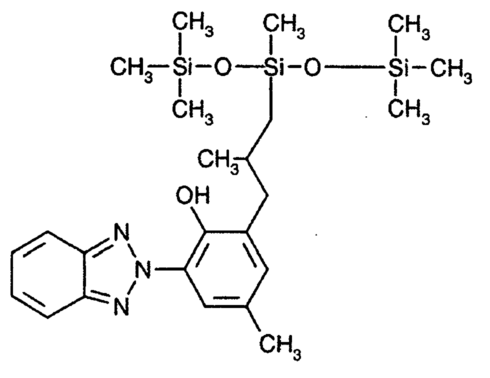

7. Composition according to any one of Claims 1 to 6, **characterized in that** the silicon derivatives with a benzotriazole functional group are present at a content ranging from 0.5% to 15%, preferably from 1% to 10%, by weight with respect to the total weight of the composition.

8. Composition according to any one of Claims 1 to 7, **characterized in that** the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

9. Composition according to Claim 8, **characterized in that** the dibenzoylmethane derivative is 4-(tert-butyl)-4'-methoxydibenzoylmethane.

10. Composition according to Claim 8, **characterized in that** the dibenzoylmethane derivative is 4-isopropyldibenzoylmethane.

11. Composition according to any one of Claims 1 to 10, where the dibenzoylmethane derivative is present at contents ranging from 0.5 to 15% by weight and preferably from 1% to 10% by weight and more particularly from 2 to 8% by weight with respect to the total weight of the composition.

12. Composition according to any one of Claims 1 to 11, where the 4,4-diarylbutadiene compound corresponds to the following formula (III): 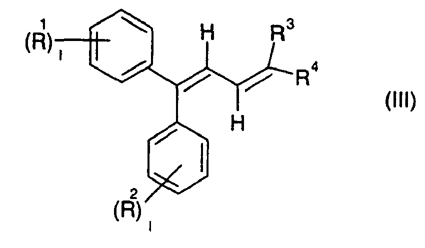 in which the diene system has the Z,Z; Z,E; E,Z or E,E configuration or is composed of mixtures of the said configurations, and where:
- R¹ and R², which are identical or different, denote hydrogen; a linear or branched C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a linear or branched C₁-C₂₀ alkoxycarbonyl radical; a linear or branched C₁-C₁₂ monoalkylamino radical; a linear or branched di(C₁-C₁₂)alkylamino radical; an aryl; a heteroaryl or a water-solubilizing substituent chosen from a carboxylate group, a sulphonate group or an ammonium residue;
- R³ denotes a COOR⁵, COR⁵, CONR⁵R⁶ or CN group; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; a C₆-C₁₈ aryl; or a C₃-C₇ heteroaryl;
- R⁴ denotes a COOR⁶, COR⁶, CONR⁵R⁶ or CN group; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- R⁵ and R⁶, which are identical or different, denote hydrogen; [V]ₒ-R⁷; C₁-C₆-alkylene-SO₃U; C₁-C₆-alkylene-PO₃U; C₁-C₆-alkylene-N(R⁸)₃⁺A⁻; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- V denotes a -CH₂-CH₂-W-, -CH₂CH₂CH₂W-, -CH(CH₃)-CH₂-W-, -CH₂-CH₂-CH₂-CH₂-W- or -CH₂-CH(CH₂CH₃)-W- group;
- A denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- W denotes O or NH;
- R⁷ and R⁸, which are identical or different, denote hydrogen; a linear or branched C₁-C₆ alkyl radical; a linear or branched C₂-C₆ alkenyl radical; or a linear or branched C₁-C₆ acyl radical;
- R⁹ denotes hydrogen; a linear or branched C₁-C₆ alkyl radical; or a C₂-C₆ alkenyl radical;
- 1 varies from 1 to 3;
- o varies from 1 to 150.

13. Composition according to Claim 12, where the compound of formula (III) is chosen from those of following formula (IIIa): 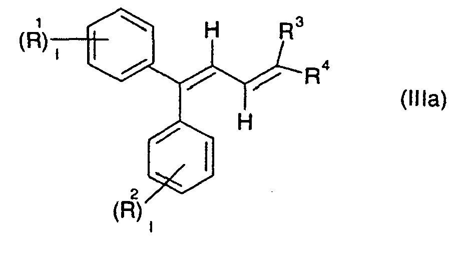 in which the diene system has the Z,Z; Z,E; E,Z or E,E configuration or is composed of mixtures of the said configurations, and where:
- R¹ and R², which are identical or different, denote hydrogen; a C₁-C₈ alkyl radical; a C₁-C₈ alkoxy radical; or a water-solubilizing substituent chosen from a carboxylate group, a sulphonate group or an ammonium residue;
- R³ denotes a COOR⁵, CONR⁵R⁶ or CN group;
- R⁴ denotes a COOR⁶ or CONR⁵R⁶ group;
- R⁵ denotes hydrogen; [V]ₒ-R⁷; C₁-C₆-alkylene-SO₃U; or C₁-C₆-alkylene-N (R⁸)₃⁺A⁻;
- R⁶ denotes [V]ₒ-R⁷; C₁-C₆-alkylene-SO₃U; or C₁-C₆₋alkylene-N (R⁸)₃⁺A⁻;
- V denotes a -CH₂-CH₂-O-, -CH₂CH₂CH₂O- or -CH(CH₃)-CH₂-O-group;
- A denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- R⁷, R⁸ and R⁹, which are identical or different, denote hydrogen or a linear or branched C₁-C₃ alkyl radical;
- l varies from 1 to 3;
- o varies from 0 to 50.

14. Composition according to Claim 13, where the compound of formula (III) is chosen from those of following formula (IIIb): 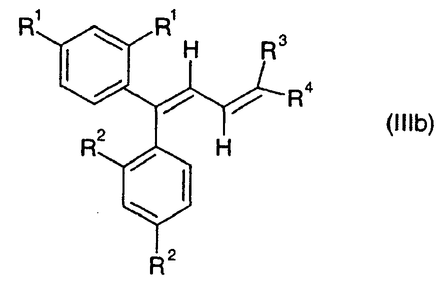 in which the diene system has the Z,Z; Z,E; E,Z or E,E configuration or is composed of mixtures of the said configurations, and where:
- R¹ and R², which are identical or different, denote hydrogen; a C₁-C₈ alkyl radical; or a C₁-C₈ alkoxy radical;
- R³ denotes a COOR⁵, CONR⁵R⁶ or CN group;
- R⁴ denotes a COOR⁶ or CONR⁵R⁶ group;
- R⁵ denotes hydrogen; [V]ₒ-R⁷; C₁-C₆-alkylene-SO₃U; or C₁-C₆-alkylene-N (R⁸)₃⁺A⁻;
- R⁶ denotes [V]ₒ-R⁷; C₁-C₆-alkylene-SO₃U; or C₁-C₆₋alkylene-N(R⁸)₃⁺A⁻;
- V denotes a -CH₂-CH₂-O-, -CH₂CH₂CH₂O- or -CH(CH₃)-CH₂-O-group;
- A denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- R⁷, R⁸ and R⁹, which are identical or different, denote hydrogen or a linear or branched C₁-C₃ alkyl radical;
- o varies from 0 to 50.

15. Composition according to Claim 14, where the compound of formula (III) is chosen from those corresponding to the following formula (IIIc): 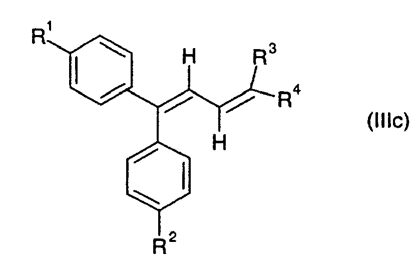 in which the diene system has the Z,Z; Z,E; E,Z or E,E configuration or is composed of mixtures of the said configurations, and where:
- R¹ and R², which are identical or different, denote hydrogen; a C₁-C₈ alkyl radical; or a C₁-C₈ alkoxy radical;
- R³ denotes a COOR⁵, CONR⁵R⁶ or CN group;
- R⁴ denotes a COOR⁶ or CONR⁵R⁶ group;
- R⁵ denotes hydrogen; [V]ₒ-R⁷; C₁-C₆-alkylene-SO₃U; or C₁-C₆-alkylene-N (R⁸)₃⁺A⁻;
- R⁶ denotes [V]ₒ-R⁷; C₁-C₆-alkylene-SO₃U; or C₁-C₆₋alkylene-N (R⁸)₃⁺A⁻;
- V denotes a -CH₂-CH₂-O-, -CH₂CH₂CH₂O- or -CH(CH₃)-CH₂-O-group;
- A denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- R⁷, R⁸ and R⁹, which are identical or different, denote hydrogen or a linear or branched C₁-C₃ alkyl radical;
- o varies from 0 to 50.

16. Composition according to Claim 12, where the 4,4-diarylbutadiene compound is chosen from the following compounds:

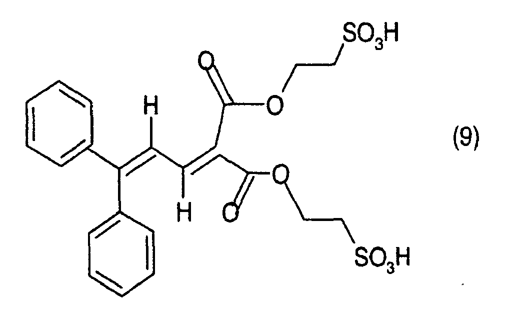

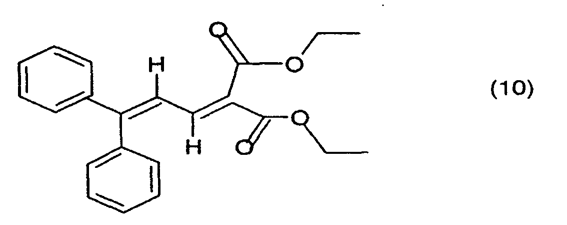

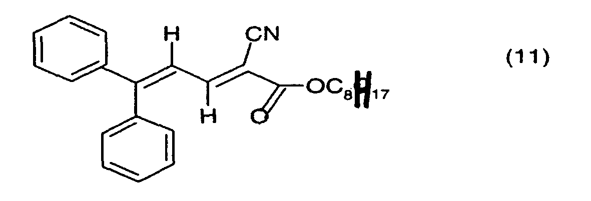

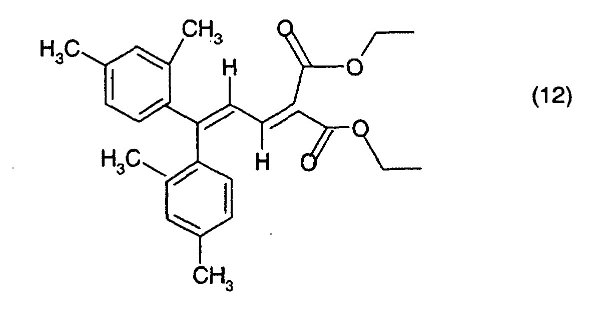

17. Composition according to any one of Claims 1 to 11, where the 4,4-diarylbutadiene compound is an oligomer corresponding to the following formula (IV): 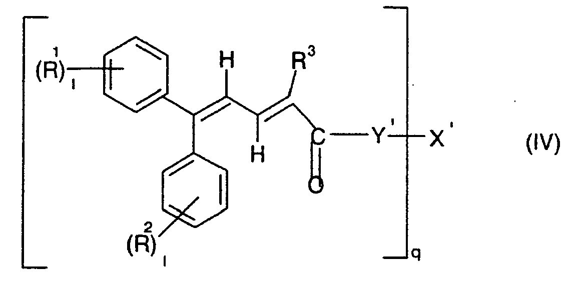 in which the diene system has the Z,Z; Z,E; E,Z or E,E configuration or is composed of mixtures of the said configurations, and where:
- R¹, R², R³ and 1 have the same meanings indicated in the formula (III) in Claim 11;
- Y' denotes an -O- or -NR¹⁰- group;
- R¹⁰ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- X' denotes a linear or branched, aliphatic or cycloaliphatic, polyol residue comprising 2 to 10 hydroxyl groups and with a valency of q; it being possible for the carbonaceous chain of the said residue to be interrupted by one or more sulphur or oxygen atoms; one or more imine groups; or one or more C₁-C₄ alkylimine groups;
- q varies from 2 to 10.

18. Composition according to Claim 17, where the compound of formula (IV) is chosen from those for which:
- R¹ and R², which are identical or different, denote hydrogen; a C₁-C₁₂ alkyl radical; a C₁-C₈ alkoxy radical; or a water-solubilizing substituent chosen from a carboxylate group, a sulphonate group or an ammonium residue;
- R³ denotes a COOR⁵, CONR⁵R⁶ or CN group; a C₃-C₁₀ cycloalkyl radical; or a C₇-C₁₀ bicycloalkyl radical;
- R⁵ and R⁶, which are identical or different, denote a linear or branched C₁-C₂₀ alkyl radical; a C₃-C₁₀ cycloalkyl radical; a C₇-C₁₀ bicycloalkyl radical; or an optionally substituted naphthyl or phenyl;
- X' denotes a polyol residue comprising from 2 to 6 hydroxyl groups and more particularly from 2 to 4.

19. Composition according to Claim 18, where the compound of formula (IV) is chosen from those for which:
- X' denotes an ethanol or pentaerythritol residue.

20. Composition according to Claim 19, where the compound of formula (IV) is chosen from the following compounds:

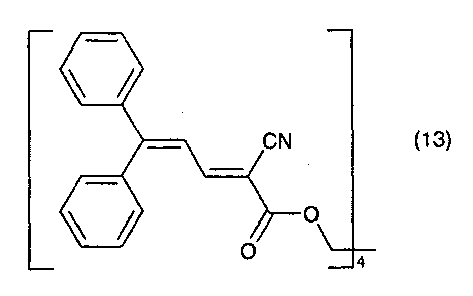

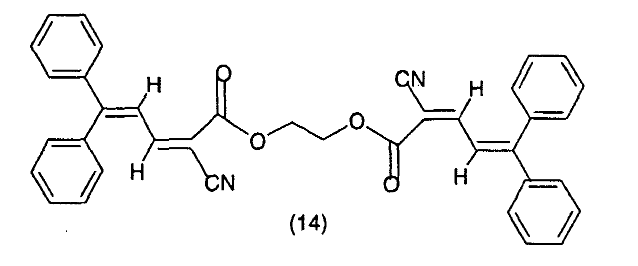

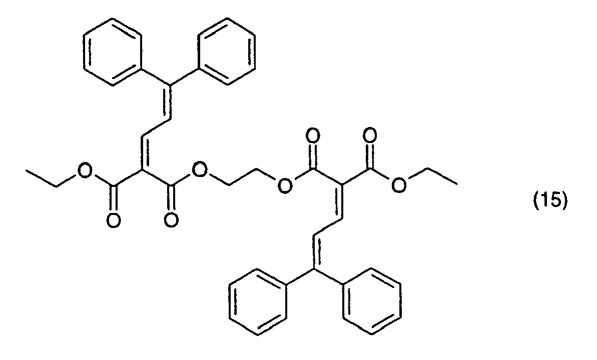

21. Composition according to any one of Claims 1 to 20, **characterized in that** the 4,4'-diarylbutadiene compound is present at contents ranging from 0.5 to 15% by weight and preferably from 1% to 10% by weight and more particularly from 2 to 8% by weight with respect to the total weight of the composition.

22. Composition according to any one of Claims 1 to 21, **characterized in that** it additionally comprises other organic screening agents which are active in the UV-A and/or UV-B regions.

23. Composition according to Claim 22, where the additional organic UV screening agent or agents are chosen from anthranilates; cinnamic derivatives; salicylic derivatives; camphor derivatives other than p-methylbenzylidenecamphor; triazine derivatives, benzophenone derivatives; β,β-diphenylacrylate derivatives; benzotriazole derivatives; benzalmalonate derivatives; benzimidazole derivatives; imidazolines; bisbenzoazolyl derivatives; p-aminobenzoic acid (PABA) derivatives; methylenebis(hydroxyphenylbenzotriazole) derivatives; screening polymers and screening silicones other than benzotriazole silicon derivatives; or dimers derived from α-alkylstyrene.

24. Composition according to Claim 23, **characterized in that** the organic UV screening agent or agents are chosen from the following compounds:
- Ethylhexyl Salicylate,
- Ethylhexyl Methoxycinnamate,
- Octocrylene,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetrasulfonate,
- Anisotriazine,
- Ethylhexyl triazone,
- Diethylhexyl Butamido Triazone,
- 2,4,6-Tris(diisobutyl 4'-aminobenzalmalonate)-s-triazine,
- Methylene bis-Benzotriazolyl Tetramethylbutylphenol,
and their mixtures.

25. Composition according to any one of Claims 1 to 24, **characterized in that** it additionally comprises coated or uncoated metal oxide pigments or nanopigments.

26. Composition according to Claim 25, **characterized in that** the said pigments or nanopigments are chosen from coated or uncoated titanium oxide, zinc oxide, iron oxide, zirconium oxide or cerium oxide, and their mixtures.

27. Composition according to any one of Claims 1 to 26, **characterized in that** it additionally comprises at least one agent for the artificial tanning and/or browning of the skin.

28. Composition according to any one of Claims 1 to 27, **characterized in that** it additionally comprises at least one adjuvant chosen from fatty substances, organic solvents, ionic or non-ionic thickeners, softeners, antioxidants, agents for combating free radicals, opacifiers, stabilizers, emollients, silicones, α-hydroxy acids, antifoaming agents, moisturizing agents, vitamins, insect repellents, fragrances, preservatives, surfactants, antiinflammatories, substance P antagonists, fillers, polymers, propellants, basifying or acidifying agents, or colorants.

29. Composition according to any one of Claims 1 to 28, **characterized in that** it is a protective composition for the human epidermis or an antisun composition and **in that** it is provided in the form of a non-ionic vesicular dispersion, of an emulsion, in particular of an emulsion of oil-in-water type, of a cream, of a milk, of a gel, of a cream gel, of a suspension, of a dispersion, of a powder, of a solid tube, of a foam or of a spray.

30. Composition according to any one of Claims 1 to 29, **characterized in that** it is a composition for making up the eyelashes, eyebrows or skin and **in that** it is provided in the anhydrous or aqueous, pasty or solid form, in the form of an emulsion, of a suspension or of a dispersion.

31. Composition according to any one of Claims 1 to 29, **characterized in that** it is a composition intended for the protection of the hair against ultraviolet rays and **in that** it is provided in the form of a shampoo, of a lotion, of a gel, of an emulsion or of a non-ionic vesicular dispersion.

32. Use of a composition as defined in Claims 1 to 29 in the manufacture of dermatological compositions intended for the protection of the skin and/or hair against ultraviolet radiation, in particular solar radiation.

33. Use of a 4,4-diarylbutadiene compound as defined in the preceding claims in the manufacture of dermatological compositions intended for the protection of the skin and/or hair against ultraviolet radiation, in particular solar radiation, comprising at least one screening agent of the benzotriazole silicon derivative type and one screening agent of the dibenzoylmethane derivative type, for the purpose of producing a synergistic effect with regard to the sun protection factors conferred.

34. Use of a cosmetic composition as defined in Claims 1 to 29 for the protection of the skin and/or hair against ultraviolet radiation, in particular solar radiation.

35. Use of a cosmetic composition comprising a 4,4-diarylbutadiene compound for the protection of the skin and/or hair against ultraviolet radiation, in particular solar radiation, comprising at least one screening agent of the benzotriazole silicon derivative type and one screening agent of the dibenzoylmethane derivative type, for the purpose of producing a synergistic effect with regard to the sun protection factors conferred.

## Patentansprüche

1. Kosmetische Zusammensetzung, insbesondere kosmetische Zusammensetzung zum Lichtschutz, **dadurch gekennzeichnet, dass** sie in einem kosmetisch akzeptablen Träger enthält:
(i) mindestens ein siliciumhaltiges Derivat mit Benzotriazolfunktion als erstes Filter;
(ii) mindestens ein Dibenzoylmethanderivat als zweites Filter; und
(iii) mindestens ein 4,4-Diarylbutadien als drittes Filter.

2. Zusammensetzung nach Anspruch 1, wobei das erste, zweite und dritte Filter in der Zusammensetzung in einem solchen Mengenanteil enthalten sind, dass bezüglich der Lichtschutzfaktoren eine synergistische Wirkung eintritt.

3. Zusammensetzung nach Anspruch 1 oder 2, wobei das siliciumhaltige Derivat mit Benzotriazolfunktion unter den Silanen und/oder Polyorganosiloxanen mit Benzotriazolfunktion ausgewählt ist, die mindestens eine Einheit der folgenden Formel (1) enthalten:
O_{(3-a)/2} Si (R₇)ₐ - G (1),
worin bedeuten:
- R₇ eine C₁₋₁₀-Alkylgruppe, die gegebenenfalls halogeniert ist, eine Phenylgruppe oder eine Trimethylsilyloxygruppe,
- a 0 oder eine ganze Zahl von 1 bis 3, und
- G eine einwertige Gruppe, die direkt an ein Siliciumatom gebunden ist und der folgenden Formel (2) entspricht: 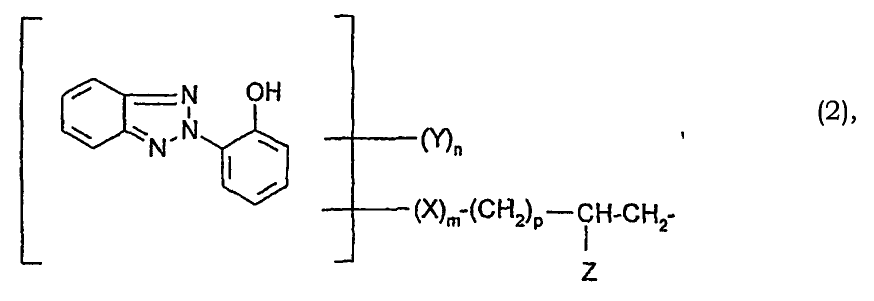
worin bedeuten:
- die Gruppen Y, die gleich oder verschieden sind, sind unter den C₁₋₈-Alkylgruppen, Halogenen und C₁₋₄-Alkoxygruppen ausgewählt, mit der Maßgabe, dass in dem zuletzt genannten Fall zwei benachbarte Gruppen Y an dem gleichen aromatischen Ring gemeinsam eine Alkylidendioxygruppe bilden können, worin die Alkylidengruppe 1 bis 2 Kohlenstoffatome aufweist,
- X O oder NH,
- Z Wasserstoff oder C₁₋₄-Alkyl,
- n 0 oder eine ganze Zahl von 1 bis 3,
- m 0 oder 1 ,
- p eine ganze Zahl von 1 bis 10.

4. Zusammensetzung nach einem der Anspruch 1 bis 3, **dadurch gekennzeichnet, dass** das siliciumhaltige Derivate mit Benzotriazolfunktion einer der folgenden Formeln (5) oder (6) entspricht: 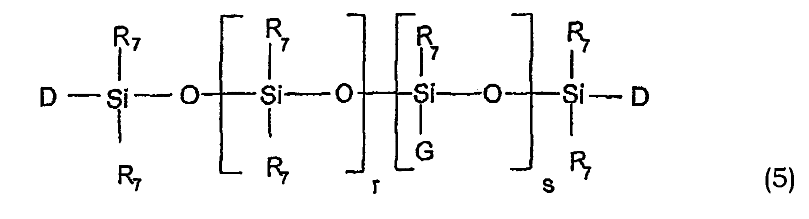 oder 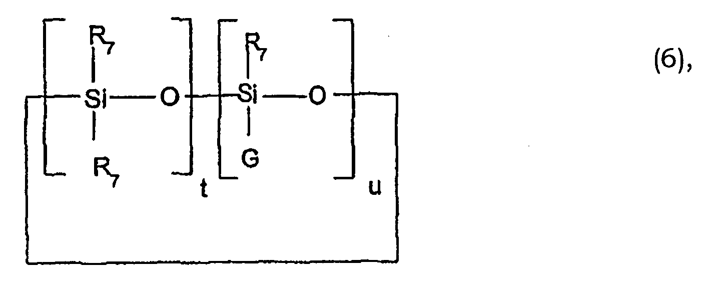 worin bedeuten:
- die Gruppen R₇, die gleich oder verschieden sind, C₁₋₁₀-Alkylgruppen, Phenyl, 3,3,3-Trifluorpropyl und Trimethylsilyloxy,
wobei mindestens 80 % der Anzahl der Gruppen R₇ Methyl bedeuten,
- die Gruppen D, die gleich oder verschieden sind, Gruppen R₇ und die Gruppe G,
- r 0 oder eine ganze Zahl von 1 bis 50 und s 0 oder eine ganze Zahl von 1 bis 20, wobei mindestens eine der Gruppen D G bedeutet, wenn s 0 ist,
- u eine ganze Zahl von 1 bis 6 und t 0 oder eine ganze Zahl von 1 bis 10, mit der Maßgabe, dass t + u mindestens 3 bedeutet, und
- das Symbol G die in Anspruch 2 für die Formel (2) angegebene Bedeutung.

5. Zusammensetzung nach Anspruch 4, **dadurch gekennzeichnet, dass** das siliciumhaltige Derivat mit Benzotriazolfunktion der folgenden Formel (7) entspricht: 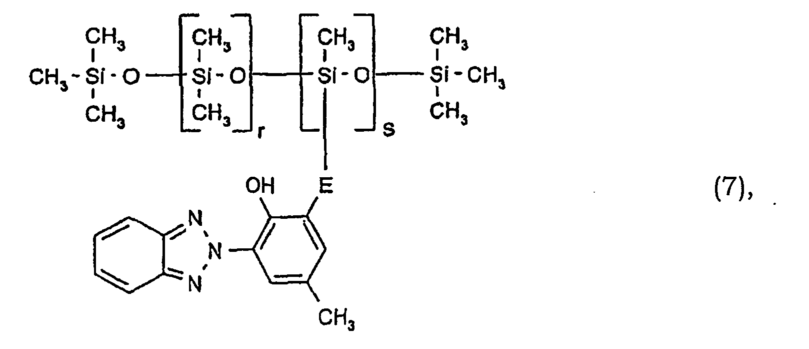 mit 0 ≤ r ≤ 10,
1 ≤ s ≤ 10;
wobei E die folgende zweiwertige Gruppe ist:

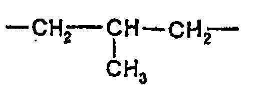

6. Zusammensetzung nach Anspruch 5, **dadurch gekennzeichnet, dass** das siliciumhaltige Derivat mit Benzotriazolfunktion das Drometrizole Trisiloxane der folgenden Formel ist:

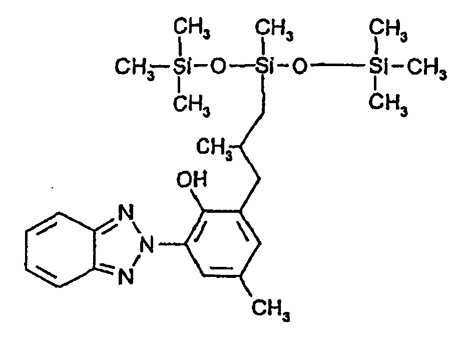

7. Zusammensetzung nach einem der Ansprüche 1 bis 6, **dadurch gekennzeichnet, dass** die siliciumhaltigen Derivate mit Benzotriazolfunktion in einer Menge von 0,5 bis 15 % und vorzugsweise 1 bis 10 Gew.-%, bezogen auf das Gesamtgewicht der Zusammensetzung, enthalten sind.

8. Zusammensetzung nach einem der Ansprüche 1 bis 7, **dadurch gekennzeichnet, dass** das Dibenzoylmethanderivat ausgewählt ist unter:
- 2-Methyldibenzoylmethan,
- 4-Methyldibenzoylmethan,
- 4-Isopropyldibenzoylmethan,
- 4-t-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Dimethoxydibenzoylmethan,
- 4-t-Butyl-4'-methoxydibenzoylmethan,
- 2-Methyl-5-isopropyl-4'-methoxydibenzoylmethan,
- 2-Methyl-5-t-butyl-4'-methoxydibenzoylmethan,
- 2,4-Dimethyl-4'-methoxydibenzoylmethan,
- 2,6-Dimethyl-4-*t*-butyl-4'-methoxydibenzoylmethan.

9. Zusammensetzung nach Anspruch 8, **dadurch gekennzeichnet, dass** das Dibenzoylmethanderivat das 4-(t-Butyl)-4'-methoxy-dibenzoylmethan ist.

10. Zusammensetzung nach Anspruch 8, **dadurch gekennzeichnet, dass** das Dibenzoylmethanderivat das 4-Isopropyldibenzoylmethan ist.

11. Zusammensetzung nach einem der Ansprüche 1 bis 10, wobei das Dibenzoylmethanderivat in einer Menge von 0,5 bis 15 Gew.-% und vorzugsweise 1 bis 10 Gew.-%, insbesondere 2 bis 8 Gew.-%, bezogen auf das Gesamtgewicht der Zusammensetzung, enthalten ist.

12. Zusammensetzung nach einem der Ansprüche 1 bis 11, wobei das 4,4-Diarylbutadien der folgenden Formel (III) entspricht: 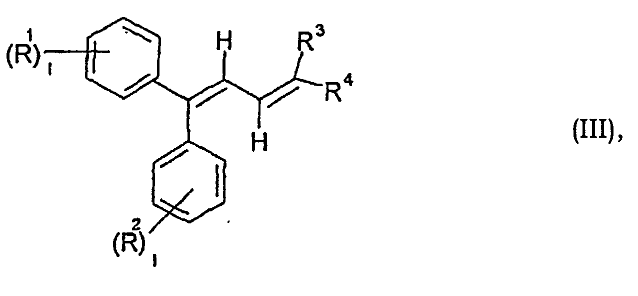 wobei das Diensystem die Konfiguration Z,Z; Z,E; E,Z; E,Z oder E,E hat oder Gemische dieser Konfigurationen bedeutet und worin bedeuten:
- R¹ und R², die gleich oder verschieden sind, Wasserstoff, eine geradkettige oder verzweigte C₁₋₂₀-Alkylgruppe; C₂₋₁₀-Alkenyl; C₁₋₁₂-Alkoxy; C₃₋₁₀-Cycloalkyl; C₃₋₁₀-Cycloalkenyl; eine geradkettige oder verzweigte C₁₋₂₀-Alkoxycarbonylgruppe; eine geradkettige oder verzweigte C₁₋₁₂-Monoalkylaminogruppe; eine geradkettige oder verzweigte C₁₋₁₂-Dialkylaminogruppe; Aryl; Heteroaryl oder einen wasserlöslichen Substituenten, der unter Carboxylat, Sulfonat oder Ammonium ausgewählt ist;
- R³ eine Gruppe COOR⁵; COR⁵; CONR⁵R⁶; CN; eine geradkettige oder verzweigte C₁₋₂₀-Alkylgruppe; C₂₋₁₀-Alkenyl; C₃₋₁₀₋Cycloalkyl; C₇₋₁₀-Bicycloalkyl; C₃₋₁₀-Cycloalkenyl; C₇₋₁₀-Bicycloalkenyl; C₆₋₁₈-Aryl; C₃₋₇-Heteroaryl;
- R⁴ eine Gruppe COOR⁶; COR⁶; CONR⁵R⁶; CN; eine geradkettige oder verzweigte C₁₋₂₀-Alkylgruppe; C₂₋₁₀-Alkenyl; C₃₋₁₀₋Cycloalkyl; C₇₋₁₀-Bicycloalkyl; C₃₋₁₀-Cycloalkenyl; C₇₋₁₀-Bicycloalkenyl; Aryl; Heteroaryl;
- R⁵ und R⁶, die gleich oder verschieden sind, Wasserstoff; [V]₀-R⁷; C₁₋₆-Alkylen-SO₃U; C₁₋₆-Alkylen-PO₃U; C₁₋₆-Alkylen-N(R⁸)₃⁺A⁻; eine geradkettige oder verzweigte C₁₋₂₀-Alkylgruppe; C₂₋₁₀-Alkenyl; C₃₋₁₀-Cycloalkyl; C₇₋₁₀-Bicycloalkyl; C₃₋₁₀-Cycloalkenyl; C₇₋₁₀-Bicycloalkenyl; Aryl; Heteroaryl;
- V eine Gruppe -CH₂-CH₂-W-, -CH₂CH₂CH₂W-, -CH(CH₃)-CH₂-W-, -CH₂-CH₂-CH₂-CH₂-W-, -CH₂-CH(CH₂CH₃)-W-;
- A Cl, Br, I, SO₄R⁹;
- U Wasserstoff, Na⁺, K⁺, Mg²⁺, Ca²⁺, Li⁺, Al³⁺, -N(R⁸)₄⁺;
- W O oder NH;
- die Gruppen R⁷ und R⁸, die gleich oder verschieden sind, Wasserstoff, eine geradkettige oder verzweigte C₁₋₆-Alkylgruppe; eine geradkettige oder verzweigte C₂₋₆-Alkenylgruppe; eine geradkettige oder verzweigte C₁₋₆-Acylgruppe;
- R⁹ Wasserstoff, eine geradkettige oder verzweigte C₁₋₆-Alkylgruppe; C₂₋₆-Alkenyl;
- 1 liegt im Bereich von 1 bis 3;
- o liegt im Bereich von 1 bis 150.

13. Zusammensetzung nach Anspruch 12, wobei die Verbindung der Formel (III) unter den Verbindungen der folgenden Formel (IIIa) ausgewählt ist: 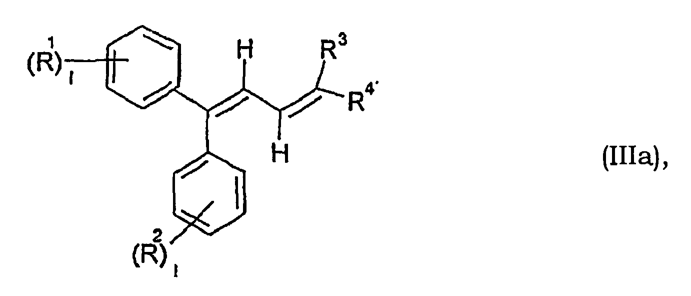 wobei das Diensystem die Konfiguration Z,Z; Z,E; E,Z oder E,E hat oder Gemische dieser Konfigurationen bedeutet und worin bedeuten:
- R¹ und R², die gleich oder verschieden sind, Wasserstoff; C₁₋₈₋Alkyl; C₁₋₈-Alkoxy; einen wasserlöslichen Substituenten, der unter Carboxylat, Sulfonat oder Ammonium ausgewählt ist;
- R³ eine Gruppe COOR⁵; CONR⁵R⁶; CN;
- R⁴ eine Gruppe COOR⁶; CONR⁵R⁶;
- R⁵ Wasserstoff; [V]₀-R⁷; C₁₋₆-Alkylen-SO₃U; C₁₋₆-Alkylen-N(R⁸)₃⁺A⁻;
- R⁶ [V]⁰-R⁷; C₁₋₆-Alkylen-SO₃U; C₁₋₆-Alkylen-N(R⁸)₃⁺A⁻;
- V eine Gruppe -CH₂-CH₂-O-, -CH₂CH₂CH₂O-, -CH(CH₃)-CH₂-O-,
- A Cl, Br, I, SO₄R⁹;
- U Wasserstoff, Na⁺, K⁺, Mg²⁺, Ca²⁺, Li⁺, Al³⁺, -N(R⁸)₄⁺
- R⁷, R⁸ und R⁹, die gleich oder verschieden sind, Wasserstoff, eine geradkettige oder verzweigte C₁₋₃-Alkylgruppe;
- l 1 bis 3;
- o 0 bis 50.

14. Zusammensetzung nach Anspruch 13, wobei die Verbindung der Formel (III) unter den Verbindungen der folgenden Formel (IIIb) ausgewählt ist: 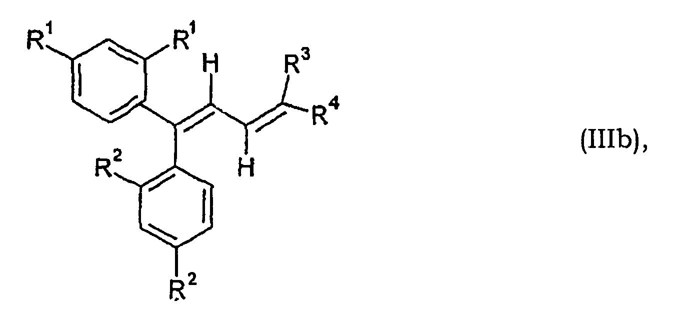 wobei das Diensystem die Konfiguration Z,Z; Z,E; E,Z oder E,E hat oder Gemische dieser Konfigurationen bedeutet und worin bedeuten:
- R¹ und R², die gleich oder verschieden sind, Wasserstoff; C₁₋₈₋Alkyl; C₁₋₈-Alkoxy;
- R³ eine Gruppe COOR⁵; CONR⁵R⁶; CN;
- R⁴ eine Gruppe COOR⁶; CONR⁵R⁶;
- R⁵ Wasserstoff; [V]₀-R⁷; C₁₋₆-Alkylen-SO₃U; C₁₋₆-Alkylen-N(R⁸)₃⁺A⁻;
- R⁶ [V]⁰-R⁷; C₁₋₆-Alkylen-SO₃U; C₁₋₆-Alkylen-N(R⁸)₃⁺A⁻;
- V eine Gruppe -CH₂-CH₂-O-, -CH₂CH₂CH₂O-, -CH(CH₃)-CH₂-O-,
- A Cl, Br, I, SO₄R⁹;
- U Wasserstoff, Na⁺, K⁺, Mg²⁺, Ca²⁺, Li⁺, Al³⁺, -N(R⁸)₄⁺
- R⁷, R⁸ und R⁹, die gleich oder verschieden sind, Wasserstoff, eine geradkettige oder verzweigte C₁₋₃-Alkylgruppe;
- o 0 bis 50.

15. Zusammensetzung nach Anspruch 14, wobei die Verbindung der Formel (III) unter den Verbindungen der folgenden Formel (IIIc) ausgewählt ist: 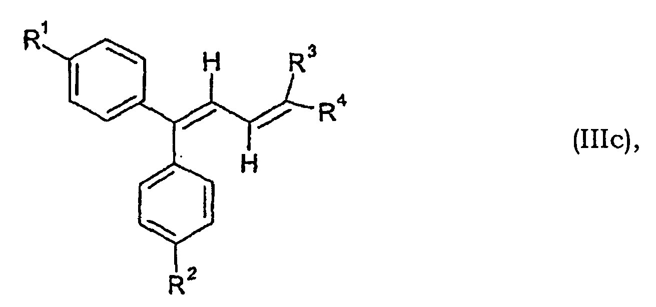 wobei das Diensystem die Konfiguration Z,Z; Z,E; E,Z oder E,E hat oder Gemische dieser Konfigurationen bedeutet und worin bedeuten:
- R¹ und R², die gleich oder verschieden sind, Wasserstoff, C₁₋₈₋Alkyl; C₁₋₈-Alkoxy;
- R³ eine Gruppe COOR⁵; CONR⁵R⁶; CN;
- R⁴ eine Gruppe COOR⁶; CONR⁵R⁶;
- R⁵ Wasserstoff; [V]₀-R⁷; C₁₋₆-Alkylen-SO₃U; C₁₋₆-Alkylen-N(R⁸)₃⁺A⁻;
- R⁶ [V]₀-R⁷; C₁₋₆-Alkylen-SO₃U; C₁₋₆-Alkylen-N(R⁸)₃⁺A⁻;
- V eine Gruppe -CH₂-CH₂-O-, -CH₂CH₂CH₂O-, -CH(CH₃)-CH₂-O-,
- A Cl, Br, I, SO₄R⁹;
- U Wasserstoff, Na⁺, K⁺, Mg²⁺, Ca²⁺, Li⁺, Al³⁺, -N(R⁸)₄⁺,
- R⁷, R⁸ und R⁹, die gleich oder verschieden sind, Wasserstoff, eine geradkettige oder verzweigte C₁₋₃-Alkylgruppe;
- o 0 bis 50.

16. Zusammensetzung nach Anspruch 12, wobei das 4,4-Diarylbutadien unter den folgenden Verbindungen ausgewählt ist:

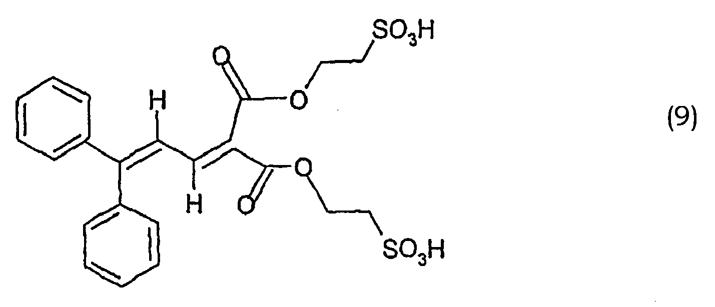

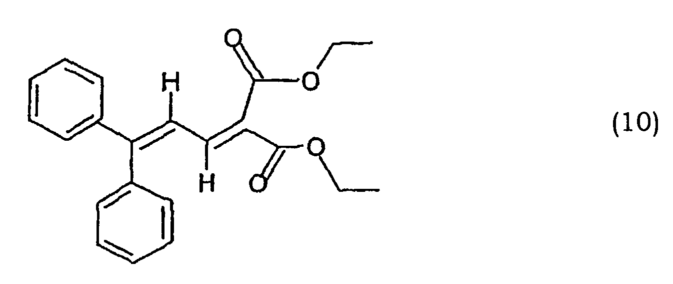

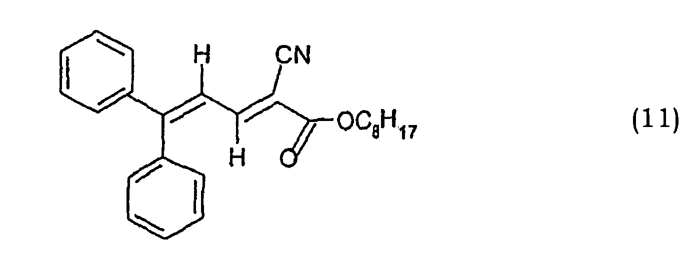

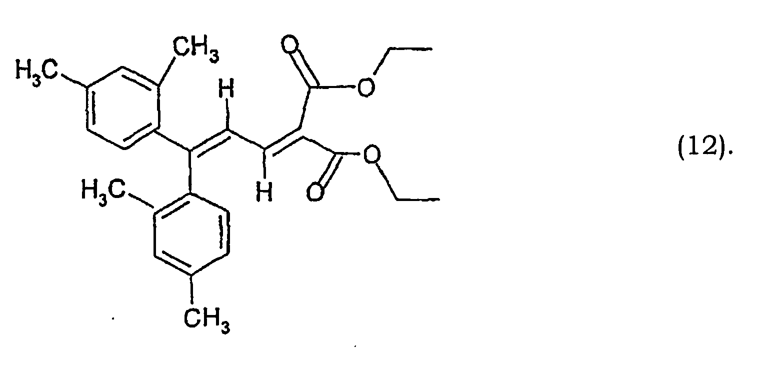

17. Zusammensetzung nach einem der Ansprüche 1 bis 11, wobei das 4,4-Diarylbutadien ein Oligomer der folgenden Formel (IV) ist: 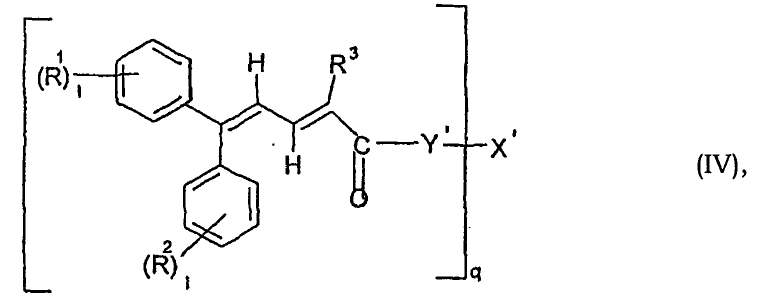 wobei das Diensystem die Konfiguration Z,Z; Z,E; E,Z oder E,E hat oder Gemische dieser Konfigurationen bedeutet und worin bedeuten:
- R¹, R², R³ und 1, die oben für die Formel (III) in Anspruch 11 angegebenen Bedeutungen;
- Y' eine Gruppe -O- oder eine Gruppe -NR¹⁰-
- R¹⁰ Wasserstoff; eine geradkettige oder verzweigte C₁₋₂₀-Alkylgruppe; C₂₋₁₀-Alkenyl; C₃₋₁₀-Cycloalkyl; C₇₋₁₀-Bicycloalkyl; C₃₋₁₀-Cycloalkenyl; C₇₋₁₀-Bicycloalkenyl; Aryl; Heteroaryl;
- X' einen geradkettigen oder verzweigten, aliphatischen oder cycloaliphatischen Polyolrest mit 2 bis 10 Hydroxygruppen und der Valenz q; wobei die Kohlenstoffkette dieses Restes durch ein oder mehrere Schwefelatome oder Sauerstoffatome unterbrochen sein kann; eine oder mehrere Imingruppen oder eine oder mehrere Alkyliminogruppen mit 1 bis 4 Kohlenstoffatomen;
- q 2 bis 10.

18. Zusammensetzung nach Anspruch 17, wobei die Verbindung der Formel (IV) unter den Verbindungen ausgewählt ist, worin bedeuten:
- R¹ und R², die gleich oder verschieden sind, Wasserstoff, C₁₋₂₁-Alkyl; C₁₋₈-Alkoxy; einen wasserlöslichen Substituenten, der unter Carboxylat, Sulfonat oder Ammonium ausgewählt ist;
- R₃ eine Gruppe COOR⁵; CONR⁵R⁶; CN; C₃₋₁₀-Cycloalkyl; C₇₋₁₀₋Bicycloalkyl;
- R⁵ und R⁶, die gleich oder verschieden sind, eine geradkettige oder verzweigte C₁₋₂₀-Alkylgruppe; C₃₋₁₀-Cycloalkyl; C₇₋₁₀-Bicycloalkyl; Naphthyl oder Phenyl, die gegebenenfalls substituiert sind;
- X' einen Polyolrest mit 2 bis 6 Hydroxygruppen und insbesondere 2 bis 4 Hydroxygruppen.

19. Zusammensetzung nach Anspruch 18, wobei die Verbindung der Formel (IV) unter den Verbindungen ausgewählt ist, worin bedeuten:
- X' einen Ethanolrest oder Pentaaerythritrest.

20. Zusammensetzung nach Anspruch 19, wobei die Verbindung der Formel (IV) unter den folgenden Verbindungen ausgewählt ist:

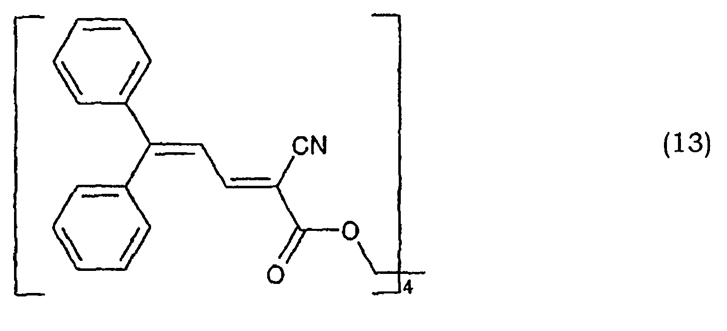

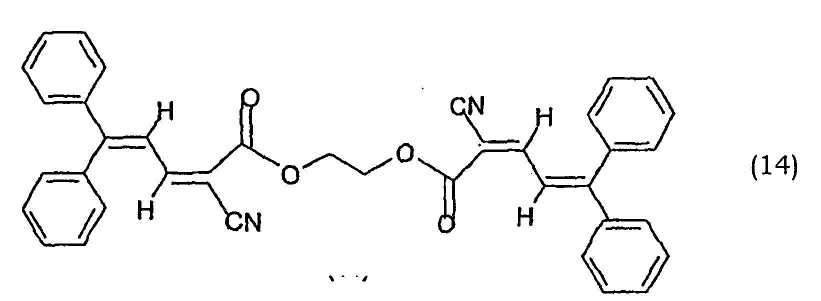

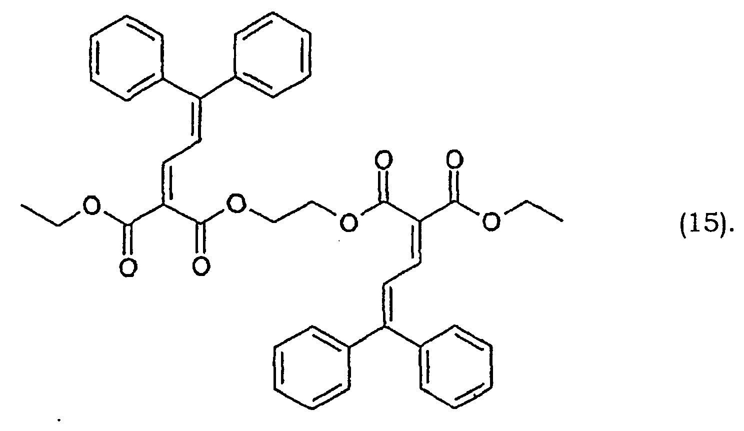

21. Zusammensetzung nach einem der Ansprüche 1 bis 20, **dadurch gekennzeichnet, dass** das 4,4-Diarylbutadien in Mengenanteilen von 0,5 bis 15 Gew.-%, vorzugsweise 1 bis 10 Gew.-% und besonders bevorzugt 2 bis 8 Gew.-%, bezogen auf das Gesamtgewicht der Zusammensetzung, enthalten ist.

22. Zusammensetzung nach einem der Ansprüche 1 bis 21, **dadurch gekennzeichnet, dass** sie ferner weitere im UV-A- und/oder UV-B-Bereich wirksame organische Filter enthält.

23. Zusammensetzung nach Anspruch 22, wobei das oder die zusätzliche(n) organische(n) UV-Filter unter den folgenden Verbindungen ausgewählt sind: Anthranilaten; Zimtsäurederivaten; Salicylsäurederivaten, Campherderivaten, die von p-Methylbenzylidencampher verschieden sind; Triazinderivaten; Benzophenonderivaten; β,β'-Diphenylacrylatderivaten, Benzotriazolderivaten; Benzalmalonatderivaten; Benzimidazolderivaten; Imidazolinen; Bis-benzoazolylderivaten; p-Aminobenzoesäurederivaten (PABA); Methylen-bis(hydroxyphenylbenzotriazol)-derivaten; polymeren Filtern und Siliconfiltern, die von den siliciumhaltigen Benzotriazolderivaten verschieden sind; von α-Alkylstyrol abgeleiteten Dimeren.

24. Zusammensetzung nach Anspruch 23, **dadurch gekennzeichnet, dass** das oder die organische(n) UV-Filter unter den folgenden Verbindungen ausgewählt sind:
- Ethylhexyl Salicylate,
- Ethylhexyl Methoxycinnamate,
- Octocrylene,
- Phenylbenzimidazole Sulfonic Acid,
- Benzophenone-3,
- Benzophenone-4,
- Benzophenone-5,
- 4-Methylbenylidene Camphor,
- Disodium Phenyl Dibenzimidazole Tetrasulfonate,
- Anisotriazine,
- Ethylhexyl Triazone,
- Diethylhexyl Butamido Triazone,
- 2,4,6-Tris(diisobutyl-4'-aminobenzalmalonat)-s-triazin,
- Methylene Bis-Benzotriazolyl Tetramethylbutylphenol
und ihren Gemischen.

25. Zusammensetzung nach einem der Ansprüche 1 bis 24, **dadurch gekennzeichnet, dass** sie ferner Pigmente oder Nanopigmente von Metalloxiden enthält, die gegebenenfalls umhüllt sind.

26. Zusammensetzung nach Anspruch 25, wobei die Pigmente oder Nanopigmente unter den Oxiden von Titan, Zink, Eisen, Zirconium, Cer und deren Gemischen ausgewählt sind, wobei sie gegebenenfalls umhüllt sind.

27. Zusammensetzung nach einem der Ansprüche 1 bis 26, **dadurch gekennzeichnet, dass** sie ferner mindestens ein Bräunungsmittel und/ oder Mittel zur künstlichen Braunfärbung der Haut enthält.

28. Zusammensetzung nach einem der Ansprüche 1 bis 27, **dadurch gekennzeichnet, dass** sie ferner mindestens einen Zusatzstoff enthält, der unter den Fettsubstanzen, organischen Lösungsmitteln, ionischen oder nichtionischen Verdickungsmitteln, beruhigenden Stoffen, Antioxidantien, Radikalfängern für freie Radikale, Trübungsmitteln, Stabilisatoren, Emollientien, Siliconen, α-Hydroxysäuren, Schaumverhütungsmitteln, Hydratisierungsmitteln, Vitaminen, insektenabwehrenden Wirkstoffen, Parfums, Konservierungsmitteln, grenzflächenaktiven Stoffen, entzündungshemmenden Stoffen, Substanz P-Antagonisten, Füllstoffen, Polymeren, Treibmitteln, Alkalisierungsmitteln, Ansäuerungsmitteln und Farbmitteln ausgewählt ist.

29. Zusammensetzung nach einem der Ansprüche 1 bis 28, **dadurch gekennzeichnet, dass** es sich um eine Zusammensetzung zum Schutz der menschlichen Epidermis oder ein Sonnenschutzmittel handelt und die Zusammensetzung als nichtionische Vesikeldispersion, Emulsion und insbesondere Emulsion vom Öl-in-Wasser-Typ, Creme, Milch, Gel, Gelcreme, Suspension, Dispersion, Pulver, fester Stift, Schaum oder Spray vorliegt.

30. Zusammensetzung nach einem der Ansprüche 1 bis 29, **dadurch gekennzeichnet, dass** es sich um eine Zusammensetzung zum Schminken der Wimpern, der Augenbrauen oder der Haut handelt und sie in fester oder pastöser, wasserfreier oder wässriger Form, als Emulsion, Suspension oder Dispersion vorliegt.

31. Zusammensetzung nach einem der Ansprüche 1 bis 29, **dadurch gekennzeichnet, dass** es sich um eine Zusammensetzung handelt, die für den Schutz der Haare gegen UV-Strahlung vorgesehen ist und die als Haarwaschmittel, Lotion, Gel, Emulsion, nichtionische Vesikeldispersion vorliegt.

32. Verwendung einer Zusammensetzung nach einem der Ansprüche 1 bis 29 für die Herstellung von dermatologischen Zusammensetzungen, die für den Schutz der Haut und/oder der Haare gegen UV-Strahlung oder insbesondere gegen Sonnenlicht vorgesehen sind.

33. Verwendung eines 4,4-Diarylbutadiens nach den vorhergehenden Ansprüchen für die Herstellung von dermatologischen Zusammensetzungen, die für den Schutz der Haut und/ oder der Haare gegen UV-Strahlung und insbesondere Sonnenlicht vorgesehen sind, die mindestens ein Filter vom Typ der siliciumhaltigen Benzotriazolderivate und ein Filter vom Typ der Dibenzoylmethanderivate enthalten, um im Hinblick auf die Lichtschutzfaktoren eine synergistische Wirkung zu erzielen.

34. Verwendung einer kosmetischen Zusammensetzung nach einem der Ansprüche 1 bis 29 zum Schutz der Haut und/oder der Haare gegen UV-Strahlung und insbesondere Sonnenlicht.

35. Verwendung einer kosmetischen Zusammensetzung, die ein 4,4-Diarylbutadien enthält, zum Schutz der Haut und/oder der Haare gegen UV-Strahlung und insbesondere Sonnenlicht, wobei die Zusammensetzung mindestens ein Filter vom Typ der siliciumhaltigen Benzotriazolderivate und ein Filter vom Typ der Dibenzoylmethanderivate enthält, um im Hinblick auf die Lichtschutzfaktoren eine synergistische Wirkung zu erzielen.
